# EUROPEAN PATENT APPLICATION

(11) **EP 3 689 896 A1**
(43) Date of publication of application: **05.08.2020**
(21) Application number: 20154529.0
(22) Date of filing: 30.01.2020
(51) Int. Cl.: C07K 14/435

(54) **GRK2 MODULATING MIRNAS**

(30) Priority: 31.01.2019 EP 19154830
(71) Applicant: Universität Heidelberg, 69117 Heidelberg (DE)
(72) Inventor: Raake, Philip Wolfram Joseph, 69214 Eppelheim (DE); Katus, Hugo, 69120 Heidelberg (DE); Meinhardt, Eric, 67071 Ludwigshafen am Rhein (DE); Müller, Oliver, 24226 Heikendorf (DE); Tscheschner, Henrike, 64646 Heppenheim (DE)
(74) Representative: Altmann Stößel Dick Patentanwälte PartG mbB

(57) **Abstract**

The present invention relates to a polynucleotide comprising and/or encoding a microRNA (miRNA) decreasing G protein-coupled receptor kinase 2 (GRK2) gene expression in a host cell; and to a vector comprising said polynucleotide The present invention further relates to said polynucleotide and/or vector for use in medicine, in particular for use in treatment and/or prevention of heart failure in a subject. The present invention also relates to devices and kits comprising said polynucleotide and/or vector.

SEQUENCE LISTING

<110>  Universität Heidelberg

<120>  UH15435EP1

<130>  anti-GRK2 miRNAs for treatment of heart failure

<150>  EP19154830.4
<151>  2019-01-31

<160>  63

<170>  PatentIn version 3.5

<210>  1
<211>  21
<212>  DNA
<213>  Artificial

<220>
<223>  miRNA sequence 1

<400>  1
tagaattcca ccaagggcct g                                                21


<210>  2
<211>  21
<212>  DNA
<213>  Artificial

<220>
<223>  miRNA sequence 2

<400>  2
ctcagtggca ctcttcgaga a                                                21


<210>  3
<211>  21
<212>  DNA
<213>  Artificial

<220>
<223>  miRNA sequence 3

<400>  3
atgacatgca gacaatgaat g                                                21


<210>  4
<211>  21
<212>  DNA
<213>  Artificial

<220>
<223>  miRNA sequence 4

<400>  4
ttgagctcca cattcttcca c                                                21

<210>  5
<211>  21
<212>  DNA
<213>  Artificial

<220>
<223>  miRNA sequence 5

<400>  5
tgttcttcat cttgggcacc c                                                21


<210>  6
<211>  21
<212>  DNA
<213>  Homo sapiens

<400>  6
caggcccttg gtggaattct a                                                21


<210>  7
<211>  21
<212>  DNA
<213>  Homo sapiens

<400>  7
ttctcgaaga gtgccactga g                                                21


<210>  8
<211>  21
<212>  DNA
<213>  Homo sapiens

<400>  8
cattcattgt ctgcatgtca t                                                21


<210>  9
<211>  21
<212>  DNA
<213>  Homo sapiens

<400>  9
gtggaagaat gtggagctca a                                                21


<210>  10
<211>  21
<212>  DNA
<213>  Homo sapiens

<400>  10
gggtgcccaa gatgaagaac a                                                21


<210>  11
<211>  19
<212>  DNA
<213>  Artificial

<220>

<223>  miRNA passenger sequence 1

<400>  11
caggccctgt ggaattcta                                                    19


<210>  12
<211>  19
<212>  DNA
<213>  Artificial

<220>
<223>  miRNA passenger sequence 2

<400>  12
ttctcgaagt gccactgag                                                    19


<210>  13
<211>  19
<212>  DNA
<213>  Artificial

<220>
<223>  miRNA passenger sequence 3

<400>  13
cattcattct gcatgtcat                                                    19


<210>  14
<211>  19
<212>  DNA
<213>  Artificial

<220>
<223>  miRNA passenger sequence

<400>  14
gtggaagagt ggagctcaa                                                    19


<210>  15
<211>  19
<212>  DNA
<213>  Artificial

<220>
<223>  miRNA passenger sequence 5

<400>  15
gggtgcccga tgaagaaca                                                    19


<210>  16
<211>  59
<212>  DNA
<213>  Artificial

<220>
<223>  pre-miRNA 1

<400>  16

tagaattcca ccaagggcct ggttttggcc actgactgac caggccctgt ggaattcta 59

<210> 17
<211> 59
<212> DNA
<213> Artificial

<220>
<223> pre-miRNA 2

<400> 17
ctcagtggca ctcttcgaga agttttggcc actgactgac ttctcgaagt gccactgag 59

<210> 18
<211> 61
<212> DNA
<213> Artificial

<220>
<223> pre-miRNA 3

<400> 18
atgacatgca gacaatgaat ggttttggcc actgactgac cattcattgt ctgcatgtca 60

t 61

<210> 19
<211> 59
<212> DNA
<213> Artificial

<220>
<223> pre-miRNA 4

<400> 19
ttgagctcca cattcttcca cgttttggcc actgactgac gtggaagagt ggagctcaa 59

<210> 20
<211> 59
<212> DNA
<213> Artificial

<220>
<223> pre-miRNA 5

<400> 20
tgttcttcat cttgggcacc cgttttggcc actgactgac gggtgcccga tgaagaaca 59

<210> 21
<211> 5142
<212> DNA
<213> Artificial

<220>
<223> pSSV9.CMVenhancer.MLC260.EGFP.miGRK2503

<400> 21
tggccactcc ctctctgcgc gctcgctcgc tcactgaggc cgggcgacca aaggtcgccc 60

gacgcccggg ctttgcccgg gcggcctcag tgagcgagcg agcgcgcaga gagggagtgg 120

ccaactccat cactaggggt tccttgtagt taatgattaa cccgccatgc tacttatcta 180

cgtagccatg ctctagagca agatcactag ttattaatag taatcaatta cggggtcatt 240

agttcatagc ccatatatgg agttccgcgt tacataactt acggtaaatg gcccgcctgg 300

ctgaccgccc aacgaccccc gcccattgac gtcaataatg acgtatgttc ccatagtaac 360

gccaataggg actttccatt gacgtcaatg ggtggagtat ttacggtaaa ctgcccactt 420

ggcagtacat caagtgtatc atatgccaag tacgccccct attgacgtca atgacggtaa 480

atggcccgcc tggcattatg cccagtacat gaccttatgg gactttccta cttggcagta 540

catctacgta ttagtcatcg ctattaccat ggtgatgcgg ttttggcagt acatcaatgg 600

gcgtggatag cggtttgact cacggggatt tccaagtctc caccccattg acgtcaatgg 660

gagtttgttt tggcaccaaa atcaacggga ctttccaaaa tgtcgtaaca actccgcccc 720

atggtcgaca agcttcctgc aggcgcgccg ctctaggacc cagagcacag agcatcgttc 780

ccaggccagg ccccagccac tgtctcttta accttgaagg catttttggg tctcacgtgt 840

ccacccaggc gggtgtcgga ctttgaacgg ctcttacttc agaagaacgg catggggtgg 900

gggggcttag gtggcctctg cctcacctac aactgccaaa agtggtcatg gggttatttt 960

taaccccagg gaagaggtat ttattgttcc acagcagggg ccggccagca ggctccttgc 1020

ccaagcgcta gcatgcatta attaagtaag tatcaaggtt acaagacagg tttaaggaga 1080

ccaatagaaa ctgggcttgt cgagacagag aagactcttg cgtttctgat aggcacctat 1140

tggtcttact gacatccact ttgcctttct ctccacaggg atccgccacc atggtgagca 1200

agggcgagga gctgttcacc ggggtggtgc ccatcctggt cgagctggac ggcgacgtaa 1260

acggccacaa gttcagcgtg tccggcgagg gcgagggcga tgccacctac ggcaagctga 1320

ccctgaagtt catctgcacc accggcaagc tgcccgtgcc ctggcccacc ctcgtgacca 1380

ccctgaccta cggcgtgcag tgcttcagcc gctaccccga ccacatgaag cagcacgact 1440

tcttcaagtc cgccatgccc gaaggctacg tccaggagcg caccatcttc ttcaaggacg 1500

acggcaacta caagacccgc gccgaggtga agttcgaggg cgacaccctg gtgaaccgca 1560

tcgagctgaa gggcatcgac ttcaaggagg acggcaacat cctggggcac aagctggagt 1620

acaactacaa cagccacaac gtctatatca tggccgacaa gcagaagaac ggcatcaagg 1680

tgaacttcaa gatccgccac aacatcgagg acggcagcgt gcagctcgcc gaccactacc 1740

agcagaacac ccccatcggc gacggccccg tgctgctgcc cgacaaccac tacctgagca 1800

cccagtccgc cctgagcaaa gaccccaacg agaagcgcga tcacatggtc ctgctggagt 1860

tcgtgaccgc cgccgggatc actctcggca tggacgagct gtacgcgatc gtttaaaggg 1920

aggtagtgag tcgaccagtg gatcctggag gcttgctgaa ggctgtatgc tgtagaattc 1980

caccaagggc ctggttttgg ccactgactg accaggccct gtggaattct acaggacaca 2040

aggcctgtta ctagcactca catggaacaa atggcccaga tctaccggtg tacaagctta 2100

aggatcctcg agatatctag agtcggggcg gccggccgct tcgagcagac atgataagat 2160

acattgatga gtttggacaa accacaacta gaatgcagtg aaaaaaatgc tttatttgtg 2220

aaatttgtga tgctattgct ttatttgtaa ccattataag ctgcaataaa caagttaaca 2280

acaacaattg cattcatttt atgtttcagg ttcaggggga ggtgtgggag gttttttaaa 2340

gcaagtaaaa cctctacaaa tgtggtaaaa tcgataccta ggctagtaat aaaccggaca 2400

ttcgaaaggc tgcggtcgaa ctctagttgc tctagagcat ggctacgtag ataagtagca 2460

tggcgggtta atcattaact acaaggaacc cctagtgatg gagttggcca ctccctctct 2520

gcgcgctcgc tcgctcactg aggccgggcg accaaaggtc gcccgacgcc cgggctttgc 2580

ccgggcggcc tcagtgagcg agcgagcgcg cagagaggga gtggccagct gcattaatga 2640

atcggccaac gcgcggggag aggcggtttg cgtattgggc gctcttccgc ttcctcgctc 2700

actgactcgc tgcgctcggt cgttcggctg cggcgagcgg tatcagctca ctcaaaggcg 2760

gtaatacggt tatccacaga atcaggggat aacgcaggaa agaacatgtg agcaaaaggc 2820

cagcaaaagg ccaggaaccg taaaaaggcc gcgttgctgg cgtttttcca taggctccgc 2880

ccccctgacg agcatcacaa aaatcgacgc tcaagtcaga ggtggcgaaa cccgacagga 2940

ctataaagat accaggcgtt tccccctgga agctccctcg tgcgctctcc tgttccgacc 3000

ctgccgctta ccggatacct gtccgccttt ctcccttcgg gaagcgtggc gctttctcat 3060

agctcacgct gtaggtatct cagttcggtg taggtcgttc gctccaagct gggctgtgtg 3120

cacgaacccc ccgttcagcc cgaccgctgc gccttatccg gtaactatcg tcttgagtcc 3180

aacccggtaa gacacgactt atcgccactg gcagcagcca ctggtaacag gattagcaga 3240

gcgaggtatg taggcggtgc tacagagttc ttgaagtggt ggcctaacta cggctacact 3300

agaaggacag tatttggtat ctgcgctctg ctgaagccag ttaccttcgg aaaaagagtt 3360

ggtagctctt gatccggcaa acaaaccacc gctggtagcg gtggtttttt tgtttgcaag 3420

cagcagatta cgcgcagaaa aaaaggatct caagaagatc ctttgatctt ttctacgggg 3480

tctgacgctc agtggaacga aaactcacgt taagggattt tggtcatgag attatcaaaa 3540

aggatcttca cctagatcct tttaaattaa aaatgaagtt ttaaatcaat ctaaagtata 3600

tatgagtaaa cttggtctga cagttaccaa tgcttaatca gtgaggcacc tatctcagcg 3660

atctgtctat ttcgttcatc catagttgcc tgactccccg tcgtgtagat aactacgata 3720

cgggagggct taccatctgg ccccagtgct gcaatgatac cgcgagaccc acgctcaccg 3780

gctccagatt tatcagcaat aaaccagcca gccggaaggg ccgagcgcag aagtggtcct 3840

gcaactttat ccgcctccat ccagtctatt aattgttgcc gggaagctag agtaagtagt 3900

tcgccagtta atagtttgcg caacgttgtt gccattgcta caggcatcgt ggtgtcacgc 3960

tcgtcgtttg gtatggcttc attcagctcc ggttcccaac gatcaaggcg agttacatga 4020

tcccccatgt tgtgcaaaaa agcggttagc tccttcggtc ctccgatcgt tgtcagaagt 4080

aagttggccg cagtgttatc actcatggtt atggcagcac tgcataattc tcttactgtc 4140

atgccatccg taagatgctt ttctgtgact ggtgagtact caaccaagtc attctgagaa 4200

tagtgtatgc ggcgaccgag ttgctcttgc ccggcgtcaa tacgggataa taccgcgcca 4260

catagcagaa ctttaaaagt gctcatcatt ggaaaacgtt cttcggggcg aaaactctca 4320

aggatcttac cgctgttgag atccagttcg atgtaaccca ctcgtgcacc caactgatct 4380

tcagcatctt ttactttcac cagcgtttct gggtgagcaa aaacaggaag gcaaaatgcc 4440

gcaaaaaagg gaataagggc gacacggaaa tgttgaatac tcatactctt cctttttcaa 4500

tattattgaa gcatttatca gggttattgt ctcatgagcg gatacatatt tgaatgtatt 4560

tagaaaaata aacaaatagg ggttccgcgc acatttcccc gaaaagtgcc acctaaattg 4620

taagcgttaa tattttgtta aaattcgcgt taaatttttg ttaaatcagc tcatttttta 4680

accaataggc cgaaatcggc aaaatccctt ataaatcaaa agaatagacc gagatagggt 4740

tgagtgttgt tccagtttgg aacaagagtc cactattaaa gaacgtggac tccaacgtca 4800

aagggcgaaa aaccgtctat cagggcgatg gcccactacg tgaaccatca ccctaatcaa 4860

gttttttggg gtcgaggtgc cgtaaagcac taaatcggaa ccctaaaggg agcccccgat 4920

ttagagcttg acggggaaag ccggcgaacg tggcgagaaa ggaagggaag aaagcgaaag 4980

gagcgggcgc tagggcgctg gcaagtgtag cggtcacgct gcgcgtaacc accacacccg 5040

ccgcgcttaa tgcgccgcta cagggcgcgt cccattcgcc attcaggctg cgcaactgtt 5100

gggaagggcg atcggtgcgg gcctcttcgc tattacgcca gc 5142

<210> 22
<211> 5142
<212> DNA
<213> Artificial

<220>
<223> pSSV9.CMVenhancer.MLC260.EGFP.miGRK2636

<400> 22
tggccactcc ctctctgcgc gctcgctcgc tcactgaggc cgggcgacca aaggtcgccc 60

gacgcccggg ctttgcccgg gcggcctcag tgagcgagcg agcgcgcaga gagggagtgg 120

ccaactccat cactaggggt tccttgtagt taatgattaa cccgccatgc tacttatcta 180

cgtagccatg ctctagagca agatcactag ttattaatag taatcaatta cggggtcatt 240

agttcatagc ccatatatgg agttccgcgt tacataactt acggtaaatg gcccgcctgg 300

ctgaccgccc aacgaccccc gcccattgac gtcaataatg acgtatgttc ccatagtaac 360

gccaataggg actttccatt gacgtcaatg ggtggagtat ttacggtaaa ctgcccactt 420

ggcagtacat caagtgtatc atatgccaag tacgccccct attgacgtca atgacggtaa 480

atggcccgcc tggcattatg cccagtacat gaccttatgg gactttccta cttggcagta 540

catctacgta ttagtcatcg ctattaccat ggtgatgcgg ttttggcagt acatcaatgg 600

gcgtggatag cggtttgact cacggggatt tccaagtctc caccccattg acgtcaatgg 660

gagtttgttt tggcaccaaa atcaacggga ctttccaaaa tgtcgtaaca actccgcccc 720

atggtcgaca agcttcctgc aggcgcgccg ctctaggacc cagagcacag agcatcgttc 780

ccaggccagg ccccagccac tgtctcttta accttgaagg catttttggg tctcacgtgt 840

ccacccaggc gggtgtcgga ctttgaacgg ctcttacttc agaagaacgg catggggtgg 900

gggggcttag gtggcctctg cctcacctac aactgccaaa agtggtcatg gggttatttt 960

taaccccagg gaagaggtat ttattgttcc acagcagggg ccggccagca ggctccttgc 1020

ccaagcgcta gcatgcatta attaagtaag tatcaaggtt acaagacagg tttaaggaga 1080

ccaatagaaa ctgggcttgt cgagacagag aagactcttg cgtttctgat aggcacctat 1140

tggtcttact gacatccact ttgcctttct ctccacaggg atccgccacc atggtgagca 1200

agggcgagga gctgttcacc ggggtggtgc ccatcctggt cgagctggac ggcgacgtaa 1260

acggccacaa gttcagcgtg tccggcgagg gcgagggcga tgccacctac ggcaagctga 1320

ccctgaagtt catctgcacc accggcaagc tgcccgtgcc ctggcccacc ctcgtgacca 1380

ccctgaccta cggcgtgcag tgcttcagcc gctaccccga ccacatgaag cagcacgact 1440

tcttcaagtc cgccatgccc gaaggctacg tccaggagcg caccatcttc ttcaaggacg 1500

acggcaacta caagacccgc gccgaggtga agttcgaggg cgacaccctg gtgaaccgca 1560

tcgagctgaa gggcatcgac ttcaaggagg acggcaacat cctggggcac aagctggagt 1620

acaactacaa cagccacaac gtctatatca tggccgacaa gcagaagaac ggcatcaagg 1680

tgaacttcaa gatccgccac aacatcgagg acggcagcgt gcagctcgcc gaccactacc 1740

agcagaacac ccccatcggc gacggccccg tgctgctgcc cgacaaccac tacctgagca 1800

cccagtccgc cctgagcaaa gaccccaacg agaagcgcga tcacatggtc ctgctggagt 1860

tcgtgaccgc cgccgggatc actctcggca tggacgagct gtacgcgatc gtttaaaggg 1920

aggtagtgag tcgaccagtg gatcctggag gcttgctgaa ggctgtatgc tgctcagtgg 1980

cactcttcga gaagttttgg ccactgactg acttctcgaa gtgccactga gcaggacaca 2040

aggcctgtta ctagcactca catggaacaa atggcccaga tctaccggtg tacaagctta 2100

aggatcctcg agatatctag agtcggggcg gccggccgct tcgagcagac atgataagat 2160

acattgatga gtttggacaa accacaacta gaatgcagtg aaaaaaatgc tttatttgtg 2220

aaatttgtga tgctattgct ttatttgtaa ccattataag ctgcaataaa caagttaaca 2280

acaacaattg cattcatttt atgtttcagg ttcaggggga ggtgtgggag gttttttaaa 2340

gcaagtaaaa cctctacaaa tgtggtaaaa tcgataccta ggctagtaat aaaccggaca 2400

ttcgaaaggc tgcggtcgaa ctctagttgc tctagagcat ggctacgtag ataagtagca 2460

tggcgggtta atcattaact acaaggaacc cctagtgatg gagttggcca ctccctctct 2520

gcgcgctcgc tcgctcactg aggccgggcg accaaaggtc gcccgacgcc cgggctttgc 2580

ccgggcggcc tcagtgagcg agcgagcgcg cagagaggga gtggccagct gcattaatga 2640

atcggccaac gcgcggggag aggcggtttg cgtattgggc gctcttccgc ttcctcgctc 2700

actgactcgc tgcgctcggt cgttcggctg cggcgagcgg tatcagctca ctcaaaggcg 2760

gtaatacggt tatccacaga atcaggggat aacgcaggaa agaacatgtg agcaaaaggc 2820

cagcaaaagg ccaggaaccg taaaaaggcc gcgttgctgg cgtttttcca taggctccgc 2880

ccccctgacg agcatcacaa aaatcgacgc tcaagtcaga ggtggcgaaa cccgacagga 2940

ctataaagat accaggcgtt tccccctgga agctccctcg tgcgctctcc tgttccgacc 3000

ctgccgctta ccggatacct gtccgccttt ctcccttcgg gaagcgtggc gctttctcat 3060

agctcacgct gtaggtatct cagttcggtg taggtcgttc gctccaagct gggctgtgtg 3120

cacgaacccc ccgttcagcc cgaccgctgc gccttatccg gtaactatcg tcttgagtcc 3180

aacccggtaa gacacgactt atcgccactg gcagcagcca ctggtaacag gattagcaga 3240

gcgaggtatg taggcggtgc tacagagttc ttgaagtggt ggcctaacta cggctacact 3300

agaaggacag tatttggtat ctgcgctctg ctgaagccag ttaccttcgg aaaaagagtt 3360

ggtagctctt gatccggcaa acaaaccacc gctggtagcg gtggtttttt tgtttgcaag 3420

cagcagatta cgcgcagaaa aaaaggatct caagaagatc ctttgatctt ttctacgggg 3480

tctgacgctc agtggaacga aaactcacgt taagggattt tggtcatgag attatcaaaa 3540

aggatcttca cctagatcct tttaaattaa aaatgaagtt ttaaatcaat ctaaagtata 3600

tatgagtaaa cttggtctga cagttaccaa tgcttaatca gtgaggcacc tatctcagcg 3660

atctgtctat ttcgttcatc catagttgcc tgactccccg tcgtgtagat aactacgata 3720

cgggagggct taccatctgg ccccagtgct gcaatgatac cgcgagaccc acgctcaccg 3780

gctccagatt tatcagcaat aaaccagcca gccggaaggg ccgagcgcag aagtggtcct 3840

gcaactttat ccgcctccat ccagtctatt aattgttgcc gggaagctag agtaagtagt 3900

tcgccagtta atagtttgcg caacgttgtt gccattgcta caggcatcgt ggtgtcacgc 3960

tcgtcgtttg gtatggcttc attcagctcc ggttcccaac gatcaaggcg agttacatga 4020

tcccccatgt tgtgcaaaaa agcggttagc tccttcggtc ctccgatcgt tgtcagaagt 4080

aagttggccg cagtgttatc actcatggtt atggcagcac tgcataattc tcttactgtc 4140

atgccatccg taagatgctt ttctgtgact ggtgagtact caaccaagtc attctgagaa 4200

tagtgtatgc ggcgaccgag ttgctcttgc ccggcgtcaa tacgggataa taccgcgcca 4260

catagcagaa ctttaaaagt gctcatcatt ggaaaacgtt cttcggggcg aaaactctca 4320

aggatcttac cgctgttgag atccagttcg atgtaaccca ctcgtgcacc caactgatct 4380

tcagcatctt ttactttcac cagcgtttct gggtgagcaa aaacaggaag gcaaaatgcc 4440

gcaaaaaagg gaataagggc gacacggaaa tgttgaatac tcatactctt cctttttcaa 4500

tattattgaa gcatttatca gggttattgt ctcatgagcg gatacatatt tgaatgtatt 4560

tagaaaaata aacaaatagg ggttccgcgc acatttcccc gaaaagtgcc acctaaattg 4620

taagcgttaa tattttgtta aaattcgcgt taaatttttg ttaaatcagc tcatttttta 4680

accaataggc cgaaatcggc aaaatccctt ataaatcaaa agaatagacc gagatagggt 4740

tgagtgttgt tccagtttgg aacaagagtc cactattaaa gaacgtggac tccaacgtca 4800

aagggcgaaa aaccgtctat cagggcgatg gcccactacg tgaaccatca ccctaatcaa 4860

gttttttggg gtcgaggtgc cgtaaagcac taaatcggaa ccctaaaggg agcccccgat 4920

ttagagcttg acggggaaag ccggcgaacg tggcgagaaa ggaagggaag aaagcgaaag 4980

gagcgggcgc tagggcgctg gcaagtgtag cggtcacgct gcgcgtaacc accacacccg 5040

ccgcgcttaa tgcgccgcta cagggcgcgt cccattcgcc attcaggctg cgcaactgtt 5100

gggaagggcg atcggtgcgg gcctcttcgc tattacgcca gc 5142

<210> 23
<211> 5144
<212> DNA
<213> Artificial

<220>
<223> pSSV9.CMVenhancer.MLC260.EGFP.miGRK21021

<400> 23
tggccactcc ctctctgcgc gctcgctcgc tcactgaggc cgggcgacca aaggtcgccc 60

gacgcccggg ctttgcccgg gcggcctcag tgagcgagcg agcgcgcaga gagggagtgg 120

ccaactccat cactaggggt tccttgtagt taatgattaa cccgccatgc tacttatcta 180

cgtagccatg ctctagagca agatcactag ttattaatag taatcaatta cggggtcatt 240

agttcatagc ccatatatgg agttccgcgt tacataactt acggtaaatg gcccgcctgg 300

ctgaccgccc aacgaccccc gcccattgac gtcaataatg acgtatgttc ccatagtaac 360

gccaataggg actttccatt gacgtcaatg ggtggagtat ttacggtaaa ctgcccactt 420

ggcagtacat caagtgtatc atatgccaag tacgccccct attgacgtca atgacggtaa 480

atggcccgcc tggcattatg cccagtacat gaccttatgg gactttccta cttggcagta 540

catctacgta ttagtcatcg ctattaccat ggtgatgcgg ttttggcagt acatcaatgg 600

gcgtggatag cggtttgact cacggggatt tccaagtctc caccccattg acgtcaatgg 660

gagtttgttt tggcaccaaa atcaacggga ctttccaaaa tgtcgtaaca actccgcccc 720

atggtcgaca agcttcctgc aggcgcgccg ctctaggacc cagagcacag agcatcgttc 780

ccaggccagg ccccagccac tgtctcttta accttgaagg catttttggg tctcacgtgt 840

ccacccaggc gggtgtcgga ctttgaacgg ctcttacttc agaagaacgg catggggtgg 900

gggggcttag gtggcctctg cctcacctac aactgccaaa agtggtcatg gggttatttt 960

taaccccagg gaagaggtat ttattgttcc acagcagggg ccggccagca ggctccttgc 1020

ccaagcgcta gcatgcatta attaagtaag tatcaaggtt acaagacagg tttaaggaga 1080

ccaatagaaa ctgggcttgt cgagacagag aagactcttg cgtttctgat aggcacctat 1140

tggtcttact gacatccact ttgcctttct ctccacaggg atccgccacc atggtgagca 1200

agggcgagga gctgttcacc ggggtggtgc ccatcctggt cgagctggac ggcgacgtaa 1260

acggccacaa gttcagcgtg tccggcgagg gcgagggcga tgccacctac ggcaagctga 1320

ccctgaagtt catctgcacc accggcaagc tgcccgtgcc ctggcccacc ctcgtgacca 1380

ccctgaccta cggcgtgcag tgcttcagcc gctaccccga ccacatgaag cagcacgact 1440

tcttcaagtc cgccatgccc gaaggctacg tccaggagcg caccatcttc ttcaaggacg 1500

acggcaacta caagacccgc gccgaggtga agttcgaggg cgacaccctg gtgaaccgca 1560

tcgagctgaa gggcatcgac ttcaaggagg acggcaacat cctggggcac aagctggagt 1620

acaactacaa cagccacaac gtctatatca tggccgacaa gcagaagaac ggcatcaagg 1680

tgaacttcaa gatccgccac aacatcgagg acggcagcgt gcagctcgcc gaccactacc 1740

agcagaacac ccccatcggc gacggccccg tgctgctgcc cgacaaccac tacctgagca 1800

cccagtccgc cctgagcaaa gaccccaacg agaagcgcga tcacatggtc ctgctggagt 1860

tcgtgaccgc cgccgggatc actctcggca tggacgagct gtacgcgatc gtttaaaggg 1920

aggtagtgag tcgaccagtg gatcctggag gcttgctgaa ggctgtatgc tgatgacatg 1980

cagacaatga atggttttgg ccactgactg accattcatt gtctgcatgt catcaggaca 2040

caaggcctgt tactagcact cacatggaac aaatggccca gatctaccgg tgtacaagct 2100

taaggatcct cgagatatct agagtcgggg cggccggccg cttcgagcag acatgataag 2160

atacattgat gagtttggac aaaccacaac tagaatgcag tgaaaaaaat gctttatttg 2220

tgaaatttgt gatgctattg ctttatttgt aaccattata agctgcaata aacaagttaa 2280

caacaacaat tgcattcatt ttatgtttca ggttcagggg gaggtgtggg aggtttttta 2340

aagcaagtaa aacctctaca aatgtggtaa aatcgatacc taggctagta ataaaccgga 2400

cattcgaaag gctgcggtcg aactctagtt gctctagagc atggctacgt agataagtag 2460

catggcgggt taatcattaa ctacaaggaa cccctagtga tggagttggc cactccctct 2520

ctgcgcgctc gctcgctcac tgaggccggg cgaccaaagg tcgcccgacg cccgggcttt 2580

gcccgggcgg cctcagtgag cgagcgagcg cgcagagagg gagtggccag ctgcattaat 2640

gaatcggcca acgcgcgggg agaggcggtt tgcgtattgg gcgctcttcc gcttcctcgc 2700

tcactgactc gctgcgctcg gtcgttcggc tgcggcgagc ggtatcagct cactcaaagg 2760

cggtaatacg gttatccaca gaatcagggg ataacgcagg aaagaacatg tgagcaaaag 2820

gccagcaaaa ggccaggaac cgtaaaaagg ccgcgttgct ggcgtttttc cataggctcc 2880

gcccccctga cgagcatcac aaaaatcgac gctcaagtca gaggtggcga aacccgacag 2940

gactataaag ataccaggcg tttccccctg gaagctccct cgtgcgctct cctgttccga 3000

ccctgccgct taccggatac ctgtccgcct ttctcccttc gggaagcgtg gcgctttctc 3060

atagctcacg ctgtaggtat ctcagttcgg tgtaggtcgt tcgctccaag ctgggctgtg 3120

tgcacgaacc ccccgttcag cccgaccgct gcgccttatc cggtaactat cgtcttgagt 3180

ccaacccggt aagacacgac ttatcgccac tggcagcagc cactggtaac aggattagca 3240

gagcgaggta tgtaggcggt gctacagagt tcttgaagtg gtggcctaac tacggctaca 3300

ctagaaggac agtatttggt atctgcgctc tgctgaagcc agttaccttc ggaaaaagag 3360

ttggtagctc ttgatccggc aaacaaacca ccgctggtag cggtggtttt tttgtttgca 3420

agcagcagat tacgcgcaga aaaaaaggat ctcaagaaga tcctttgatc ttttctacgg 3480

ggtctgacgc tcagtggaac gaaaactcac gttaagggat tttggtcatg agattatcaa 3540

aaaggatctt cacctagatc cttttaaatt aaaaatgaag ttttaaatca atctaaagta 3600

tatatgagta aacttggtct gacagttacc aatgcttaat cagtgaggca cctatctcag 3660

cgatctgtct atttcgttca tccatagttg cctgactccc cgtcgtgtag ataactacga 3720

tacgggaggg cttaccatct ggccccagtg ctgcaatgat accgcgagac ccacgctcac 3780

cggctccaga tttatcagca ataaaccagc cagccggaag ggccgagcgc agaagtggtc 3840

ctgcaacttt atccgcctcc atccagtcta ttaattgttg ccgggaagct agagtaagta 3900

gttcgccagt taatagtttg cgcaacgttg ttgccattgc tacaggcatc gtggtgtcac 3960

gctcgtcgtt tggtatggct tcattcagct ccggttccca acgatcaagg cgagttacat 4020

gatcccccat gttgtgcaaa aaagcggtta gctccttcgg tcctccgatc gttgtcagaa 4080

gtaagttggc cgcagtgtta tcactcatgg ttatggcagc actgcataat tctcttactg 4140

tcatgccatc cgtaagatgc ttttctgtga ctggtgagta ctcaaccaag tcattctgag 4200

aatagtgtat gcggcgaccg agttgctctt gcccggcgtc aatacgggat aataccgcgc 4260

cacatagcag aactttaaaa gtgctcatca ttggaaaacg ttcttcgggg cgaaaactct 4320

caaggatctt accgctgttg agatccagtt cgatgtaacc cactcgtgca cccaactgat 4380

cttcagcatc ttttactttc accagcgttt ctgggtgagc aaaaacagga aggcaaaatg 4440

ccgcaaaaaa gggaataagg gcgacacgga aatgttgaat actcatactc ttcctttttc 4500

aatattattg aagcatttat cagggttatt gtctcatgag cggatacata tttgaatgta 4560

tttagaaaaa taaacaaata ggggttccgc gcacatttcc ccgaaaagtg ccacctaaat 4620

tgtaagcgtt aatattttgt taaaattcgc gttaaatttt tgttaaatca gctcattttt 4680

taaccaatag gccgaaatcg gcaaaatccc ttataaatca aaagaataga ccgagatagg 4740

gttgagtgtt gttccagttt ggaacaagag tccactatta aagaacgtgg actccaacgt 4800

caaagggcga aaaaccgtct atcagggcga tggcccacta cgtgaaccat caccctaatc 4860

aagttttttg gggtcgaggt gccgtaaagc actaaatcgg aaccctaaag ggagcccccg 4920

atttagagct tgacggggaa agccggcgaa cgtggcgaga aaggaaggga agaaagcgaa 4980

aggagcgggc gctagggcgc tggcaagtgt agcggtcacg ctgcgcgtaa ccaccacacc 5040

cgccgcgctt aatgcgccgc tacagggcgc gtcccattcg ccattcaggc tgcgcaactg 5100

ttgggaaggg cgatcggtgc gggcctcttc gctattacgc cagc 5144

<210> 24
<211> 5142
<212> DNA
<213> Artificial

<220>
<223> pSSV9.CMVenhancer.MLC260.EGFP.miGRK2794

<400> 24
tggccactcc ctctctgcgc gctcgctcgc tcactgaggc cgggcgacca aaggtcgccc 60

gacgcccggg ctttgcccgg gcggcctcag tgagcgagcg agcgcgcaga gagggagtgg 120

ccaactccat cactaggggt tccttgtagt taatgattaa cccgccatgc tacttatcta 180

cgtagccatg ctctagagca agatcactag ttattaatag taatcaatta cggggtcatt 240

agttcatagc ccatatatgg agttccgcgt tacataactt acggtaaatg gcccgcctgg 300

ctgaccgccc aacgaccccc gcccattgac gtcaataatg acgtatgttc ccatagtaac 360

gccaataggg actttccatt gacgtcaatg ggtggagtat ttacggtaaa ctgcccactt 420

ggcagtacat caagtgtatc atatgccaag tacgccccct attgacgtca atgacggtaa 480

atggcccgcc tggcattatg cccagtacat gaccttatgg gactttccta cttggcagta 540

catctacgta ttagtcatcg ctattaccat ggtgatgcgg ttttggcagt acatcaatgg 600

gcgtggatag cggtttgact cacggggatt tccaagtctc caccccattg acgtcaatgg 660

gagtttgttt tggcaccaaa atcaacggga ctttccaaaa tgtcgtaaca actccgcccc 720

atggtcgaca agcttcctgc aggcgcgccg ctctaggacc cagagcacag agcatcgttc 780

ccaggccagg ccccagccac tgtctcttta accttgaagg catttttggg tctcacgtgt 840

ccacccaggc gggtgtcgga ctttgaacgg ctcttacttc agaagaacgg catggggtgg 900

gggggcttag gtggcctctg cctcacctac aactgccaaa agtggtcatg gggttatttt 960

taaccccagg gaagaggtat ttattgttcc acagcagggg ccggccagca ggctccttgc 1020

ccaagcgcta gcatgcatta attaagtaag tatcaaggtt acaagacagg tttaaggaga 1080

ccaatagaaa ctgggcttgt cgagacagag aagactcttg cgtttctgat aggcacctat 1140

tggtcttact gacatccact ttgcctttct ctccacaggg atccgccacc atggtgagca 1200

agggcgagga gctgttcacc ggggtggtgc ccatcctggt cgagctggac ggcgacgtaa 1260

acggccacaa gttcagcgtg tccggcgagg gcgagggcga tgccacctac ggcaagctga 1320

ccctgaagtt catctgcacc accggcaagc tgcccgtgcc ctggcccacc ctcgtgacca 1380

ccctgaccta cggcgtgcag tgcttcagcc gctaccccga ccacatgaag cagcacgact 1440

tcttcaagtc cgccatgccc gaaggctacg tccaggagcg caccatcttc ttcaaggacg 1500

acggcaacta caagacccgc gccgaggtga agttcgaggg cgacaccctg gtgaaccgca 1560

tcgagctgaa gggcatcgac ttcaaggagg acggcaacat cctggggcac aagctggagt 1620

acaactacaa cagccacaac gtctatatca tggccgacaa gcagaagaac ggcatcaagg 1680

tgaacttcaa gatccgccac aacatcgagg acggcagcgt gcagctcgcc gaccactacc 1740

agcagaacac ccccatcggc gacggccccg tgctgctgcc cgacaaccac tacctgagca 1800

cccagtccgc cctgagcaaa gaccccaacg agaagcgcga tcacatggtc ctgctggagt 1860

tcgtgaccgc cgccgggatc actctcggca tggacgagct gtacgcgatc gtttaaaggg 1920

aggtagtgag tcgaccagtg gatcctggag gcttgctgaa ggctgtatgc tgttgagctc 1980

cacattcttc cacgttttgg ccactgactg acgtggaaga gtggagctca acaggacaca 2040

aggcctgtta ctagcactca catggaacaa atggcccaga tctaccggtg tacaagctta 2100

aggatcctcg agatatctag agtcggggcg gccggccgct tcgagcagac atgataagat 2160

acattgatga gtttggacaa accacaacta gaatgcagtg aaaaaaatgc tttatttgtg 2220

aaatttgtga tgctattgct ttatttgtaa ccattataag ctgcaataaa caagttaaca 2280

acaacaattg cattcatttt atgtttcagg ttcaggggga ggtgtgggag gttttttaaa 2340

gcaagtaaaa cctctacaaa tgtggtaaaa tcgataccta ggctagtaat aaaccggaca 2400

ttcgaaaggc tgcggtcgaa ctctagttgc tctagagcat ggctacgtag ataagtagca 2460

tggcgggtta atcattaact acaaggaacc cctagtgatg gagttggcca ctccctctct 2520

gcgcgctcgc tcgctcactg aggccgggcg accaaaggtc gcccgacgcc cgggctttgc 2580

ccgggcggcc tcagtgagcg agcgagcgcg cagagaggga gtggccagct gcattaatga 2640

atcggccaac gcgcggggag aggcggtttg cgtattgggc gctcttccgc ttcctcgctc 2700

actgactcgc tgcgctcggt cgttcggctg cggcgagcgg tatcagctca ctcaaaggcg 2760

gtaatacggt tatccacaga atcaggggat aacgcaggaa agaacatgtg agcaaaaggc 2820

cagcaaaagg ccaggaaccg taaaaaggcc gcgttgctgg cgtttttcca taggctccgc 2880

ccccctgacg agcatcacaa aaatcgacgc tcaagtcaga ggtggcgaaa cccgacagga 2940

ctataaagat accaggcgtt tccccctgga agctccctcg tgcgctctcc tgttccgacc 3000

ctgccgctta ccggatacct gtccgccttt ctcccttcgg gaagcgtggc gctttctcat 3060

agctcacgct gtaggtatct cagttcggtg taggtcgttc gctccaagct gggctgtgtg 3120

cacgaacccc ccgttcagcc cgaccgctgc gccttatccg gtaactatcg tcttgagtcc 3180

aacccggtaa gacacgactt atcgccactg gcagcagcca ctggtaacag gattagcaga 3240

gcgaggtatg taggcggtgc tacagagttc ttgaagtggt ggcctaacta cggctacact 3300

agaaggacag tatttggtat ctgcgctctg ctgaagccag ttaccttcgg aaaaagagtt 3360

ggtagctctt gatccggcaa acaaaccacc gctggtagcg gtggtttttt tgtttgcaag 3420

cagcagatta cgcgcagaaa aaaaggatct caagaagatc ctttgatctt ttctacgggg 3480

tctgacgctc agtggaacga aaactcacgt taagggattt tggtcatgag attatcaaaa 3540

aggatcttca cctagatcct tttaaattaa aaatgaagtt ttaaatcaat ctaaagtata 3600

tatgagtaaa cttggtctga cagttaccaa tgcttaatca gtgaggcacc tatctcagcg 3660

atctgtctat ttcgttcatc catagttgcc tgactccccg tcgtgtagat aactacgata 3720

cgggagggct taccatctgg ccccagtgct gcaatgatac cgcgagaccc acgctcaccg 3780

gctccagatt tatcagcaat aaaccagcca gccggaaggg ccgagcgcag aagtggtcct 3840

gcaactttat ccgcctccat ccagtctatt aattgttgcc gggaagctag agtaagtagt 3900

tcgccagtta atagtttgcg caacgttgtt gccattgcta caggcatcgt ggtgtcacgc 3960

tcgtcgtttg gtatggcttc attcagctcc ggttcccaac gatcaaggcg agttacatga 4020

tcccccatgt tgtgcaaaaa agcggttagc tccttcggtc ctccgatcgt tgtcagaagt 4080

aagttggccg cagtgttatc actcatggtt atggcagcac tgcataattc tcttactgtc 4140

atgccatccg taagatgctt ttctgtgact ggtgagtact caaccaagtc attctgagaa 4200

tagtgtatgc ggcgaccgag ttgctcttgc ccggcgtcaa tacgggataa taccgcgcca 4260

catagcagaa ctttaaaagt gctcatcatt ggaaaacgtt cttcggggcg aaaactctca 4320

aggatcttac cgctgttgag atccagttcg atgtaaccca ctcgtgcacc caactgatct 4380

tcagcatctt ttactttcac cagcgtttct gggtgagcaa aaacaggaag gcaaaatgcc 4440

gcaaaaaagg gaataagggc gacacggaaa tgttgaatac tcatactctt cctttttcaa 4500

tattattgaa gcatttatca gggttattgt ctcatgagcg gatacatatt tgaatgtatt 4560

tagaaaaata aacaaatagg ggttccgcgc acatttcccc gaaaagtgcc acctaaattg 4620

taagcgttaa tattttgtta aaattcgcgt taaatttttg ttaaatcagc tcatttttta 4680

accaataggc cgaaatcggc aaaatccctt ataaatcaaa agaatagacc gagatagggt 4740

tgagtgttgt tccagtttgg aacaagagtc cactattaaa gaacgtggac tccaacgtca 4800

aagggcgaaa aaccgtctat cagggcgatg gcccactacg tgaaccatca ccctaatcaa 4860

gttttttggg gtcgaggtgc cgtaaagcac taaatcggaa ccctaaaggg agcccccgat 4920

ttagagcttg acggggaaag ccggcgaacg tggcgagaaa ggaagggaag aaagcgaaag 4980

gagcgggcgc tagggcgctg gcaagtgtag cggtcacgct gcgcgtaacc accacacccg 5040

ccgcgcttaa tgcgccgcta cagggcgcgt cccattcgcc attcaggctg cgcaactgtt 5100

gggaagggcg atcggtgcgg gcctcttcgc tattacgcca gc 5142

<210> 25
<211> 5142
<212> DNA
<213> Artificial

<220>
<223> pSSV9.CMVenhancer.MLC260.EGFP.miGRK22245

<400> 25
tggccactcc ctctctgcgc gctcgctcgc tcactgaggc cgggcgacca aaggtcgccc 60

gacgcccggg ctttgcccgg gcggcctcag tgagcgagcg agcgcgcaga gagggagtgg 120

ccaactccat cactaggggt tccttgtagt taatgattaa cccgccatgc tacttatcta 180

cgtagccatg ctctagagca agatcactag ttattaatag taatcaatta cggggtcatt 240

agttcatagc ccatatatgg agttccgcgt tacataactt acggtaaatg gcccgcctgg 300

ctgaccgccc aacgaccccc gcccattgac gtcaataatg acgtatgttc ccatagtaac 360

gccaataggg actttccatt gacgtcaatg ggtggagtat ttacggtaaa ctgcccactt 420

ggcagtacat caagtgtatc atatgccaag tacgccccct attgacgtca atgacggtaa 480

atggcccgcc tggcattatg cccagtacat gaccttatgg gactttccta cttggcagta 540

catctacgta ttagtcatcg ctattaccat ggtgatgcgg ttttggcagt acatcaatgg 600

gcgtggatag cggtttgact cacggggatt tccaagtctc caccccattg acgtcaatgg 660

gagtttgttt tggcaccaaa atcaacggga ctttccaaaa tgtcgtaaca actccgcccc 720

atggtcgaca agcttcctgc aggcgcgccg ctctaggacc cagagcacag agcatcgttc 780

ccaggccagg ccccagccac tgtctcttta accttgaagg catttttggg tctcacgtgt 840

ccacccaggc gggtgtcgga ctttgaacgg ctcttacttc agaagaacgg catggggtgg 900

gggggcttag gtggcctctg cctcacctac aactgccaaa agtggtcatg gggttatttt 960

taaccccagg gaagaggtat ttattgttcc acagcagggg ccggccagca ggctccttgc 1020

ccaagcgcta gcatgcatta attaagtaag tatcaaggtt acaagacagg tttaaggaga 1080

ccaatagaaa ctgggcttgt cgagacagag aagactcttg cgtttctgat aggcacctat 1140

tggtcttact gacatccact ttgcctttct ctccacaggg atccgccacc atggtgagca 1200

agggcgagga gctgttcacc ggggtggtgc ccatcctggt cgagctggac ggcgacgtaa 1260

acggccacaa gttcagcgtg tccggcgagg gcgagggcga tgccacctac ggcaagctga 1320

ccctgaagtt catctgcacc accggcaagc tgcccgtgcc ctggcccacc ctcgtgacca 1380

ccctgaccta cggcgtgcag tgcttcagcc gctaccccga ccacatgaag cagcacgact 1440

tcttcaagtc cgccatgccc gaaggctacg tccaggagcg caccatcttc ttcaaggacg 1500

acggcaacta caagacccgc gccgaggtga agttcgaggg cgacaccctg gtgaaccgca 1560

tcgagctgaa gggcatcgac ttcaaggagg acggcaacat cctggggcac aagctggagt 1620

acaactacaa cagccacaac gtctatatca tggccgacaa gcagaagaac ggcatcaagg 1680

tgaacttcaa gatccgccac aacatcgagg acggcagcgt gcagctcgcc gaccactacc 1740

agcagaacac ccccatcggc gacggccccg tgctgctgcc cgacaaccac tacctgagca 1800

cccagtccgc cctgagcaaa gaccccaacg agaagcgcga tcacatggtc ctgctggagt 1860

tcgtgaccgc cgccgggatc actctcggca tggacgagct gtacgcgatc gtttaaaggg 1920

aggtagtgag tcgaccagtg gatcctggag gcttgctgaa ggctgtatgc tgtgttcttc 1980

atcttgggca cccgttttgg ccactgactg acgggtgccc gatgaagaac acaggacaca 2040

aggcctgtta ctagcactca catggaacaa atggcccaga tctaccggtg tacaagctta 2100

aggatcctcg agatatctag agtcggggcg gccggccgct tcgagcagac atgataagat 2160

acattgatga gtttggacaa accacaacta gaatgcagtg aaaaaaatgc tttatttgtg 2220

aaatttgtga tgctattgct ttatttgtaa ccattataag ctgcaataaa caagttaaca 2280

acaacaattg cattcatttt atgtttcagg ttcaggggga ggtgtgggag gttttttaaa 2340

gcaagtaaaa cctctacaaa tgtggtaaaa tcgataccta ggctagtaat aaaccggaca 2400

ttcgaaaggc tgcggtcgaa ctctagttgc tctagagcat ggctacgtag ataagtagca 2460

tggcgggtta atcattaact acaaggaacc cctagtgatg gagttggcca ctccctctct 2520

gcgcgctcgc tcgctcactg aggccgggcg accaaaggtc gcccgacgcc cgggctttgc 2580

ccgggcggcc tcagtgagcg agcgagcgcg cagagaggga gtggccagct gcattaatga 2640

atcggccaac gcgcggggag aggcggtttg cgtattgggc gctcttccgc ttcctcgctc 2700

actgactcgc tgcgctcggt cgttcggctg cggcgagcgg tatcagctca ctcaaaggcg 2760

gtaatacggt tatccacaga atcaggggat aacgcaggaa agaacatgtg agcaaaaggc 2820

cagcaaaagg ccaggaaccg taaaaaggcc gcgttgctgg cgtttttcca taggctccgc 2880

ccccctgacg agcatcacaa aaatcgacgc tcaagtcaga ggtggcgaaa cccgacagga 2940

ctataaagat accaggcgtt tccccctgga agctccctcg tgcgctctcc tgttccgacc 3000

ctgccgctta ccggatacct gtccgccttt ctcccttcgg gaagcgtggc gctttctcat 3060

agctcacgct gtaggtatct cagttcggtg taggtcgttc gctccaagct gggctgtgtg 3120

cacgaacccc ccgttcagcc cgaccgctgc gccttatccg gtaactatcg tcttgagtcc 3180

aacccggtaa gacacgactt atcgccactg gcagcagcca ctggtaacag gattagcaga 3240

gcgaggtatg taggcggtgc tacagagttc ttgaagtggt ggcctaacta cggctacact 3300

agaaggacag tatttggtat ctgcgctctg ctgaagccag ttaccttcgg aaaaagagtt 3360

ggtagctctt gatccggcaa acaaaccacc gctggtagcg gtggtttttt tgtttgcaag 3420

cagcagatta cgcgcagaaa aaaaggatct caagaagatc ctttgatctt ttctacgggg 3480

tctgacgctc agtggaacga aaactcacgt taagggattt tggtcatgag attatcaaaa 3540

aggatcttca cctagatcct tttaaattaa aaatgaagtt ttaaatcaat ctaaagtata 3600

tatgagtaaa cttggtctga cagttaccaa tgcttaatca gtgaggcacc tatctcagcg 3660

atctgtctat ttcgttcatc catagttgcc tgactccccg tcgtgtagat aactacgata 3720

cgggagggct taccatctgg ccccagtgct gcaatgatac cgcgagaccc acgctcaccg 3780

gctccagatt tatcagcaat aaaccagcca gccggaaggg ccgagcgcag aagtggtcct 3840

gcaactttat ccgcctccat ccagtctatt aattgttgcc gggaagctag agtaagtagt 3900

tcgccagtta atagtttgcg caacgttgtt gccattgcta caggcatcgt ggtgtcacgc 3960

tcgtcgtttg gtatggcttc attcagctcc ggttcccaac gatcaaggcg agttacatga 4020

tcccccatgt tgtgcaaaaa agcggttagc tccttcggtc ctccgatcgt tgtcagaagt 4080

aagttggccg cagtgttatc actcatggtt atggcagcac tgcataattc tcttactgtc 4140

atgccatccg taagatgctt ttctgtgact ggtgagtact caaccaagtc attctgagaa 4200

tagtgtatgc ggcgaccgag ttgctcttgc ccggcgtcaa tacgggataa taccgcgcca 4260

catagcagaa ctttaaaagt gctcatcatt ggaaaacgtt cttcggggcg aaaactctca 4320

aggatcttac cgctgttgag atccagttcg atgtaaccca ctcgtgcacc caactgatct 4380

tcagcatctt ttactttcac cagcgtttct gggtgagcaa aaacaggaag gcaaaatgcc 4440

gcaaaaaagg gaataagggc gacacggaaa tgttgaatac tcatactctt cctttttcaa 4500

tattattgaa gcatttatca gggttattgt ctcatgagcg gatacatatt tgaatgtatt 4560

tagaaaaata aacaaatagg ggttccgcgc acatttcccc gaaaagtgcc acctaaattg 4620

taagcgttaa tattttgtta aaattcgcgt taaatttttg ttaaatcagc tcatttttta 4680

accaataggc cgaaatcggc aaaatccctt ataaatcaaa agaatagacc gagatagggt 4740

tgagtgttgt tccagtttgg aacaagagtc cactattaaa gaacgtggac tccaacgtca 4800

aagggcgaaa aaccgtctat cagggcgatg gcccactacg tgaaccatca ccctaatcaa 4860

gttttttggg gtcgaggtgc cgtaaagcac taaatcggaa ccctaaaggg agcccccgat 4920

ttagagcttg acggggaaag ccggcgaacg tggcgagaaa ggaagggaag aaagcgaaag 4980

gagcgggcgc tagggcgctg gcaagtgtag cggtcacgct gcgcgtaacc accacacccg 5040

ccgcgcttaa tgcgccgcta cagggcgcgt cccattcgcc attcaggctg cgcaactgtt 5100

gggaagggcg atcggtgcgg gcctcttcgc tattacgcca gc 5142

<210> 26
<211> 263
<212> DNA
<213> Artificial

<220>
<223> Myosin light chain promoter

<400> 26
gacccagagc acagagcatc gttcccaggc caggccccag ccactgtctc tttaaccttg 60

aaggcatttt tgggtctcac gtgtccaccc aggcgggtgt cggactttga acggctctta 120

cttcagaaga acggcatggg gtgggggggc ttaggtggcc tctgcctcac ctacaactgc 180

caaaagtggt catggggtta tttttaaccc cagggaagag gtatttattg ttccacagca 240

ggggccggcc agcaggctcc ttg 263

<210> 27
<211> 422
<212> DNA
<213> Artificial

<220>
<223> MLP promoter + intronic element of beta-globin

<400> 27
gacccagagc acagagcatc gttcccaggc caggccccag ccactgtctc tttaaccttg 60

aaggcatttt tgggtctcac gtgtccaccc aggcgggtgt cggactttga acggctctta 120

cttcagaaga acggcatggg gtgggggggc ttaggtggcc tctgcctcac ctacaactgc 180

caaaagtggt catggggtta tttttaaccc cagggaagag gtatttattg ttccacagca 240

ggggccggcc agcaggctcc ttgcccaagc gctagcatgc attaattaag taagtatcaa 300

ggttacaaga caggtttaag gagaccaata gaaactgggc ttgtcgagac agagaagact 360

cttgcgtttc tgataggcac ctattggtct tactgacatc cactttgcct ttctctccac 420

ag 422

<210>  28
<211>  23
<212>  DNA
<213>  Homo sapiens

<400>  28
ctggaggctt gctgaaggct gta                                              23


<210>  29
<211>  41
<212>  DNA
<213>  Homo sapiens

<400>  29
acacaaggcc tgttactagc actcacatgg aacaaatggc c                          41


<210>  30
<211>  21
<212>  DNA
<213>  Mus musculus

<400>  30
attcgatgca cactgaagtc a                                                21


<210>  31
<211>  21
<212>  DNA
<213>  Mus musculus

<400>  31
agaagtcaca ggctaggccc a                                                21


<210>  32
<211>  19
<212>  DNA
<213>  Artificial

<220>
<223>  miRNA passenger sequence 6

<400>  32
tgacttcagt gcatcgaat                                                   19


<210>  33
<211>  19
<212>  DNA
<213>  Artificial

<220>
<223>  miRNA passenger sequence 7

<400>  33
tgggcctact gtgacttct                                                   19


<210>  34
<211>  32

<212>  DNA
<213>  Artificial

<220>
<223>  CMV enhacer forward primer

<400>  34
atatattaga tctgaattca cgcgtcggcg gc                                    32


<210>  35
<211>  33
<212>  DNA
<213>  Artificial

<220>
<223>  CMV enhancer reverse

<400>  35
atatattgtc gaccatgggg cggagttgtt acg                                   33


<210>  36
<211>  32
<212>  DNA
<213>  Artificial

<220>
<223>  MLC260 forward primer

<400>  36
atatattgtc gacaagcttc ctgcaggcgc gc                                    32


<210>  37
<211>  36
<212>  DNA
<213>  Artificial

<220>
<223>  MLC260 reverse primer

<400>  37
atatattgga tccctgtgga gagaaaggca aagtgg                                36


<210>  38
<211>  24
<212>  DNA
<213>  Artificial

<220>
<223>  amplification forward primer

<400>  38
gagctcgccc gacattgatt attg                                             24


<210>  39
<211>  28
<212>  DNA
<213>  Artificial

<220>
<223>  amplification reverse primer

<400>  39
gattaccggt agatctgggc catttgtt                                          28


<210>  40
<211>  23
<212>  DNA
<213>  Artificial

<220>
<223>  GRK2 forward primer

<400>  40
cgacccatgg ccgacctgga ggc                                               23


<210>  41
<211>  27
<212>  DNA
<213>  Artificial

<220>
<223>  GRK2 reverse primer

<400>  41
cagcctcgag tcagaggccg ttggcac                                           27


<210>  42
<211>  21
<212>  DNA
<213>  Artificial

<220>
<223>  miGRK2 1.4

<400>  42
attcgatgca cactgaagtc a                                                 21


<210>  43
<211>  21
<212>  DNA
<213>  Artificial

<220>
<223>  miGRK2 1.6

<400>  43
agaagtcaca ggctaggccc a                                                 21


<210>  44
<211>  21
<212>  DNA
<213>  Artificial

<220>
<223>  miR neg ctrl.

<400>  44
aaatgtactg cgcgtggaga c                                                21

<210>  45
<211>  50
<212>  DNA
<213>  Artificial

<220>
<223>  miGRK2 1.4-2

<400>  45
gtcgtatcca gtgcagggtc cgaggtattc gcactggata cgactgactt                 50

<210>  46
<211>  50
<212>  DNA
<213>  Artificial

<220>
<223>  miR neg ctrl.-2

<400>  46
gtcgtatcca gtgcagggtc cgaggtattc gcactggata cgacgtctcc                 50

<210>  47
<211>  20
<212>  DNA
<213>  Artificial

<220>
<223>  Standard Rev

<400>  47
ccagtgcagg gtccgaggta                                                  20

<210>  48
<211>  20
<212>  DNA
<213>  Artificial

<220>
<223>  miR neg ctrl. Fw

<400>  48
ctcgcaaatg tactgcgcgt                                                  20

<210>  49
<211>  18
<212>  DNA
<213>  Artificial

<220>
<223>  miGRK2 1.4 Fw

<400>  49
cgcggcattc gatgcaca                                                    18

<210>  50
<211>  20
<212>  DNA
<213>  Artificial

<220>
<223>  Grk2 Fw

<400>  50
ccctctcacc atctctgagc                                                    20


<210>  51
<211>  20
<212>  DNA
<213>  Artificial

<220>
<223>  Grk2 Rev

<400>  51
cggttgggga acaagtagaa                                                    20


<210>  52
<211>  24
<212>  DNA
<213>  Artificial

<220>
<223>  Hprt1 Fw

<400>  52
gaggagtcct gttgatgttg ccag                                               24


<210>  53
<211>  24
<212>  DNA
<213>  Artificial

<220>
<223>  Hprt1 Rev

<400>  53
ggctggccta taggctcata gtgc                                               24


<210>  54
<211>  20
<212>  DNA
<213>  Artificial

<220>
<223>  Anf Fw

<400>  54
tgccggtaga agatgaggtc                                                    20


<210>  55
<211>  20

<212>  DNA
<213>  Artificial

<220>
<223>  Anf Rev

<400>  55
tgcttttcaa gagggcagat                                                    20


<210>  56
<211>  20
<212>  DNA
<213>  Artificial

<220>
<223>  Bnp Fw

<400>  56
ctgaaggtgc tgtcccagat                                                    20


<210>  57
<211>  20
<212>  DNA
<213>  Artificial

<220>
<223>  Bnp Rev

<400>  57
ccttggtcct tcaagagctg                                                    20


<210>  58
<211>  24
<212>  DNA
<213>  Artificial

<220>
<223>  beta-Mhc Fw

<400>  58
gccaacacca acctgtccaa gttc                                               24


<210>  59
<211>  24
<212>  DNA
<213>  Artificial

<220>
<223>  beta-Mhc Rev

<400>  59
tgcaaaggct ccaggtctga gggc                                               24


<210>  60
<211>  20
<212>  DNA
<213>  Artificial

<220>
<223>  Col-1a Fw

<400>  60
gtgttcccta ctcagccgtc                                                   20


<210>  61
<211>  20
<212>  DNA
<213>  Artificial

<220>
<223>  Col-1a Rev

<400>  61
actcgaacgg gaatccatcg                                                   20


<210>  62
<211>  22
<212>  DNA
<213>  Artificial

<220>
<223>  Postn Fw

<400>  62
acttcagctc ctgtaagaac tg                                                22


<210>  63
<211>  22
<212>  DNA
<213>  Artificial

<220>
<223>  Postn Rev

<400>  63
agggcagcat tcatatagca ca                                                22

## Description

The present invention relates to a polynucleotide comprising and/or encoding a microRNA (miRNA) decreasing G protein-coupled receptor kinase 2 (GRK2) gene expression in a host cell; and to a vector comprising said polynucleotide. The present invention further relates to said polynucleotide and/or vector for use in medicine, in particular for use in treatment and/or prevention of heart failure in a subject. The present invention also relates to devices and kits comprising said polynucleotide and/or vector.

Treatment options for heart failure at present focus on alleviating symptoms and preventing progression of disease, while causative treatment usually is restricted to addressing underlying risk factors like infections, alcohol ingestion, anemia, and others. Thus, treatment may be pharmacological treatment (i.e. with Beta blockers, etc.), non-surgical device treatment (i.e. cardiac resynchronization therapy), or surgical treatment (i.e. left ventricular assist device, total artificial heart implantation, or heart transplantation). However, not all treatment options are open for all patients, e.g. heart transplantation may not be available for older patients due to contraindications stated by transplant guidelines.

More recently, gene therapeutic approaches were tested for treatment of heart failure (Greenberg et al. (2016), Lancet 387:1178); as vectors, adeno-associated virus (AAV) vectors are frequently used, with different genotypes having different efficacy in transferring genetic material to the heart (Bish et al. (2008), Human Gene Therapy 19:1359; Naso et al. (2017), BioDrugs 31:317). Moreover, chimeric and mutant AAV strains were used (Asokan (2012, Mol Ther 20(4):699).

The protein GRK2 was found to be overexpressed in heart muscle cells of patients suffering from heart failure (Ungerer et al, (1993), Circulation 87, 454-463), which is believed to contribute to hypertrophic remodeling and to a decreased response to catecholamines (Evron et al. (2012), Trends Pharmacol. Sei. 33, 154-164; Cannavo et al. (2013), Front Physiol 4, 264). In consequence, it could be shown that a heart specific knockout of GRK2 in mice decreases mortality and improves recovery after infarction (Raake et al., (2008), Eur. Heart J. 34, 1437-1447).

There is, nonetheless, a need in the art to provide improved means and methods for treatment of heart failure. In particular, there is a need to provide means and methods avoiding at least in part the drawbacks of the prior art as discussed above.

This problem is solved by the subject matter of the present invention with the features of the independent claims. Preferred embodiments, which might be realized in an isolated fashion or in any arbitrary combination are listed in the dependent claims.

Accordingly, the present invention relates to a polynucleotide comprising and/or encoding a microRNA (miRNA) decreasing G protein-coupled receptor kinase 2 (GRK2) expression in a host cell.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term "essentially identical" includes 100% identity. The aforesaid applies to the term "essentially complementary" mutatis mutandis.

The term "G protein-coupled receptor kinase 2", abbreviated as "GRK2", is understood by the skilled person as relating to a protein known under this designation, which is a member of the G protein-coupled receptor kinase (GRK) family. Preferably, GRK2 is a mammalian GRK2, more preferably a primate or pig GRK2; most preferably GRK2 is a human GRK2. Preferably, GRK2 has an amino acid sequence as shown in Genbank Acc No. NP_001231807.1 or a sequence at least 50%, more preferably at least 75%, even more preferably at least 85%, still more preferably at least 90%, still more preferably at least 95%, identical to said sequence; most preferably, GRK2 has an amino acid sequence as shown in Genbank Acc No. NP_001231807.1. Preferably, the GRK2 is encoded by an mRNA having the sequence as shown in Genbank Acc No. NM_001244878.1 or a sequence at least 50%, more preferably at least 75%, even more preferably at least 85%, still more preferably at least 90%, still more preferably at least 95%, identical to said sequence. Most preferably, the GRK2 is encoded by an mRNA having the sequence as shown in Genbank Acc No. NM_001244878.1. Also preferably, GRK2 has an amino acid sequence as shown in Genbank Acc No. NP_001610.2, or a sequence at least 50%, more preferably at least 75%, even more preferably at least 85%, still more preferably at least 90%, still more preferably at least 95%, identical to said sequence; most preferably, the GRK2 has an amino acid sequence as shown in Genbank Acc No. NP_001610.2. Preferably, the GRK2 is encoded by an mRNA having the sequence as shown in Genbank Acc No. NM_001619.5 or a sequence at least 50%, more preferably at least 75%, even more preferably at least 85%, still more preferably at least 90%, still more preferably at least 95%, identical to said sequence; most preferably, the GRK2 is encoded by an mRNA having the sequence as shown in Genbank Acc No. NM_001619.5. Correspondingly, the terms "GRK2 target nucleic acid sequence" and "GRK2 target sequence", as used herein, relate to a sequence comprised in an mRNA encoding a GRK2 which, when targeted with a miRNA, causes concentration of a GRK2 in a host cell to decrease and/or to be lower than in the absence of such miRNA. Preferably, the GRK2 target sequence consists of at least 15, preferably of from 18 to 22, more preferably 21, contiguous nucleotides of a nucleic acid sequence at least 70% identical to a GRK2 encoding polynucleotide, preferably a GRK2 encoding mRNA, preferably as specified above. More preferably, the GRK2 target sequence consists of at least 15, preferably of from 18 to 22, more preferably 21, contiguous nucleotides of a nucleic acid sequence of a GRK2 encoding polynucleotide, preferably of a GRK2 encoding mRNA. Most preferably, the GRK2 target sequence comprises, preferably consists of, a sequence of any one of SEQ ID NOs:6 to 10.

The terms "microRNA" and "miRNA" are understood by the skilled person to relate to an RNA molecule comprising, preferably consisting of, at least 15, preferably of from 18 to 22, more preferably 19, 20, 21, or 22, most preferably 21 nucleotides being reverse complementary to an mRNA. Preferably, the sequence of the miRNA is at least 90% identical to the reverse complement of the target sequence, more preferably at least 95%, most preferably 100%. The miRNA of the present invention decreases G protein-coupled receptor kinase 2 (GRK2) expression in a host cell, wherein said GRK2 is the GRK2 as specified herein above. Thus, preferably, the miRNA comprises, preferably consists of, a nucleic acid sequence (miRNA sequence) being the reverse complement of a GRK2 target nucleic acid sequence (GRK2 target sequence) as specified herein. Preferably, the miRNA comprises, more preferably consists of, a nucleic acid sequence selected from SEQ ID NOs:1 to 5 or a sequence at least 80%, more preferably at least 90%, still more preferably at least 95%, identical thereto; most preferably the miRNA consists of a nucleic acid sequence selected from SEQ ID NOs:1 to 5. It is, however, also envisaged that the miRNA comprises, more preferably consists of, a nucleic acid sequence selected from SEQ ID NOs:30 or 31 or a sequence at least 80% identical thereto; more preferably the miRNA consists of a nucleic acid sequence selected from SEQ ID NOs:30 or 31. Preferably, the miRNA is comprised in an RNA comprising further sequences, more preferably in a pre-miRNA. Preferably, the miRNA is comprised in an RNA comprising said miRNA sequence, a spacer sequence, and a passenger sequence, wherein said passenger sequence is the reverse complement of said miRNA sequence but lacks at least one nucleotide corresponding to a position of from 4 to 12 of said reverse complement of said miRNA sequence; more preferably, the passenger sequence lacks at least the nucleotides, preferably lacks the nucleotides, corresponding to positions 9 and 10 of said reverse complement of said miRNA sequence. Preferably, said passenger sequence is selected from SEQ ID NOs:11 to 15 or a sequence at least 80% identical thereto; preferably the passenger sequence comprises a nucleic acid sequence selected from SEQ ID NOs:11 to 15. Also preferably, said passenger sequence is selected from SEQ ID NOs:32 and 33 or a sequence at least 80% identical thereto; preferably the passenger sequence comprises a nucleic acid sequence selected from SEQ ID NOs:32 and 33. Preferably, the miRNA is comprised in an RNA comprising the nucleic acid sequences of (i) SEQ ID NOs:1 and 11, (ii) SEQ ID NOs:2 and 12, (iii) SEQ ID NOs:3 and 13, (iv) SEQ ID NOs:4 and 14, (v) SEQ ID NOs:5 and 15, or (vi) any combination of (i) to (v). More preferably, the miRNA is comprised in an RNA comprising a nucleic acid sequence selected from SEQ ID NOs:16 to 20 or a sequence at least 80%, more preferably at least 90%, still more preferably at least 95%, identical thereto; even more preferably comprising, most preferably consisting of a nucleic acid sequence selected from SEQ ID NOs:16 to 20.

The term "polynucleotide" is understood by the skilled person and, preferably, refers to a linear or circular nucleic acid molecule. Preferably, the polynucleotide is RNA or is DNA, including cDNA. The term encompasses single as well as partially or completely doublestranded polynucleotides. Preferably, the polynucleotide comprises a stem-loop structure, in which the passenger sequence and the miRNA are comprised in the stem structure. In accordance with the above, the term "polyribonucleotide" relates to a polynucleotide consisting of ribonucleotides, i.e. preferably, is RNA. As will be understood by the skilled person, biosynthesis of RNA incorporates uridine ribonucleotides instead of thymidine ribonucleotides; thus, in polyribonucleotides synthesized by biosynthesis, the "T" residues in the sequences indicated herein would be replaced by "U" residues. The polynucleotide comprises or encodes the miRNA as specified elsewhere herein. Preferably, the polynucleotide comprises the miRNA as specified elsewhere herein; thus, preferably, the polynucleotide is an RNA; more preferably, the polynucleotide encodes the miRNA as specified elsewhere herein; thus, preferably, the polynucleotide is an RNA or, more preferably, is DNA.

Preferably, the polynucleotide is an RNA comprising further sequences, more preferably a pre-miRNA; or the polynucleotide encodes an RNA comprising further sequences, more preferably encodes a pre-miRNA. Preferably, said RNA comprises said miRNA sequence, a spacer sequence, and a passenger sequence, wherein said passenger sequence is the passenger sequence as specified herein above. Preferably, said RNA being or encoded by the polynucleotide comprises the nucleic acid sequences of (i) SEQ ID NOs:1 and 11, (ii) SEQ ID NOs:2 and 12, (iii) SEQ ID NOs:3 and 13, (iv) SEQ ID NOs:4 and 14, (v) SEQ ID NOs:5 and 15, or (vi) any combination of (i) to (v). More preferably, said RNA being or encoded by the polynucleotide comprises a nucleic acid sequence selected from SEQ ID NOs:16 to 20 or a sequence at least 80% identical thereto; even more preferably comprises, most preferably consists of a nucleic acid sequence selected from SEQ ID NOs:16 to 20. Preferably, the polynucleotide comprises further sequences, in particular 5' and 3' flaking sequences derived from a naturally occurring miRNA, preferably from miRNA-155, miRNA-30, or miRNA-33, more preferably miRNA-155. Preferably, said 5' flanking sequence has the sequence of SEQ ID NO:28 and/or said 3' flanking sequence has the sequence of SEQ ID NO:29.

Preferably, the polynucleotide further comprises a promoter causing expression of at least a part of said polynucleotide in a host cell, preferably a myosin light chain promoter or active fragment thereof. Thus, preferably, the polynucleotide is an expression construct. More preferably, the polynucleotide further comprises the sequence of any one of SEQ ID NOs:26 to 27. Also preferably, the polynucleotide comprises, preferably consists of, the nucleic acid sequence of any one of SEQ ID NOs:21 to 25 or a sequence at least 50%, more preferably at least 75%, even more preferably at least 85%, still more preferably at least 90%, even more preferably at least 95%, identical to said sequence, preferably comprises the nucleic acid sequence of any one of SEQ ID NOs: 21 to 25.

Preferably, the polynucleotide comprises or encodes more than one miRNA; thus, preferably, the polynucleotide comprises or encodes at least one further miRNA. As will be understood by the skilled person, said at least further miRNA may be identical to the first miRNA or may be non-identical to said first miRNA. Thus, preferably, the further miRNA targets the identical GRK2 target sequence as the first miRNA, a non-identical GRK2 target sequence, or a non-GRK2 target sequence. In the latter case, the further miRNA preferably targets a further target known or suspected to be relevant for a disease or disorder as specified herein.

Also preferably, the polynucleotide is a vector, preferably an expression vector. The term "vector", preferably, encompasses phage, plasmid, viral or retroviral vectors as well artificial chromosomes, such as bacterial or yeast artificial chromosomes. Moreover, the term also relates to targeting constructs which allow for random or site-directed integration of the targeting construct into genomic DNA. Such targeting constructs, preferably, comprise DNA of sufficient length for either homologous or heterologous recombination as described in detail below. The vector encompassing the polynucleotide of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerenes. Alternatively, a plasmid vector may be introduced by heat shock or electroporation techniques. Should the vector be a virus, it may be packaged in vitro using an appropriate packaging cell line or alternative packaging systems prior to application to host cells. More preferably, in the vector, the sequence encoding a miRNA is operatively linked to expression control sequences allowing expression in eukaryotic cells or isolated fractions thereof. Expression of said polynucleotide comprises transcription of the polynucleotide. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in eukaryotic host cells are those specified elsewhere herein or the AOX1 or GAL1 promoter in yeast or the CMV-, SV40-, RSV-promoter (Rous sarcoma virus), CMV-enhancer, SV40-enhancer or a globin intron in mammalian and other animal cells. Preferably, the regulatory element comprises the AAV p40 promoter, more preferably a promoter as specified herein above. Moreover, inducible expression control sequences may be used in an expression vector encompassed by the present invention. Suitable expression control sequences are well known in the art. Beside elements which are responsible for the initiation of transcription such regulatory elements may also comprise transcription termination signals, such as the SV40-poly-A site, the tk-poly-A site, or the AAV poly-A site downstream of the polynucleotide. Methods which can be used to construct the polynucleotides of the invention are well known to those skilled in the art.

Preferably, the polynucleotide further comprises at least one sequence mediating packaging of said polynucleotide into a viral particle; thus, preferably, the polynucleotide can preferably be provided packaged into a viral particle. Preferably, said viral particle is a replication-incompetent viral particle, e.g. a virus-like particle (VLP). Packaging sequences for relevant viruses are well-known in the art. Preferably, the virus is an adeno-associated virus (AAV), an adenovirus, a retrovirus, preferably a HIV-derivative, or a herpesvirus. Preferably, the virus is an adeno-associated virus; thus, preferably, the viral particle is an adeno-associated virus-like particle (AA-VLP). AA-VLPs are known in the art, including derivatives having a modified cellular tropism compared to wildtype AAV.

Moreover, comprised are also chemically modified polynucleotides including naturally occurring modified polynucleotides such as glycosylated or methylated polynucleotides or artificially modified derivatives such as biotinylated polynucleotides or polynucleotides comprising phosphorothioates. The polynucleotide of the present invention shall be provided, preferably, either as an isolated polynucleotide (i.e. isolated from its natural context) or in genetically modified form. Preferably, the polynucleotide has a length of at most 100 kb, more preferably at most 10 kb, even more preferably at most 5 kb, still more preferably at most 1 kb, most preferably at most 100 bp. Preferably, the polynucleotide is a non-naturally occurring polynucleotide; thus, preferably, the polynucleotide is an artificial polynucleotide. Also preferably, the polynucleotide is a chimeric polynucleotide; more preferably, the polynucleotide comprises at least one nucleic acid sequence heterologous to the remaining nucleic acid sequences it comprises.

As used herein, the term polynucleotide, preferably, includes variants of the specifically indicated polynucleotides. More preferably, the term polynucleotide relates to polynucleotides essentially identical to the specific polynucleotides indicated. Most preferably, the term polynucleotide relates to the specific polynucleotides indicated. The term "polynucleotide variant", as used herein, relates to a variant of a polynucleotide related to comprising a nucleic acid sequence characterized in that the sequence can be derived from the aforementioned specific nucleic acid sequence by at least one nucleotide substitution, addition and/or deletion, wherein the polynucleotide variant shall have the biological activity or activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof. Also preferably, said polynucleotide variant comprises a naturally occurring allele of the specific polynucleotide or of a functional subsequence thereof. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in standard textbooks. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of O.lx to 5x SSC (pH 7.2). If organic solvent is present in the abovementioned buffer, for example 50% formamide, the temperature under standard conditions is approximately 42°C. The hybridization conditions for DNA:DNA hybrids are preferably for example O.lx SSC and 20°C to 45°C, preferably between 30°C and 45°C. The hybridization conditions for DNA:RNA hybrids are preferably, for example, O.lx SSC and 30°C to 55°C, preferably between 45°C and 55°C. The abovementioned hybridization temperatures are determined for example for a nucleic acid with approximately 100 bp (= base pairs) in length and a G+C content of 50% in the absence of formamide; accordingly, other conditions more suitable for low-G+C DNA, which are in principle known to the skilled person, may be found to be more appropriate by the skilled person. The skilled worker knows how to determine the hybridization conditions required by referring to standard textbooks. Alternatively, polynucleotide variants are obtainable by PCR-based techniques such as mixed oligonucleotide primer-based amplification of DNA, e.g. using degenerated primers. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequences thereof. The percent identity values are, preferably, calculated over the entire nucleic acid sequence region of the indicated polynucleotide. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Feng & Doolittle (1987), J. Mol. Evolution 25: 351, Higgins et al. (1989), CABIOS 5:151) or the programs Gap and BestFit (Needleman and Wunsch (1970), J. Mol. Biol. 48:443) and Smith and Waterman (1981), Adv. Appl. Math. 2: 482-489), are preferably used. Preferably, said programs are used with their standard parameters. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. More preferably, the sequence identity values recited above in percent (%) are determined using the program Needle over the entire sequence region with the following settings: Matrix: DNAfull, gap open: 10, Gap extend 0.5, end gap penalty false, end gap open 10, and end gap extend 0.5.

A polynucleotide comprising a fragment of any of the specifically indicated nucleic acid sequences, said polynucleotide retaining the indicated activity or activities, is preferably also encompassed as a variant polynucleotide of the present invention. A fragment as meant herein, preferably, comprises at least 18, preferably at least 20, more preferably at least 21 consecutive nucleotides of any one of the specific nucleic acid sequences and still has the indicated activity. The polynucleotides of the present invention either consist of, essentially consist of, or comprise the aforementioned nucleic acid sequences. Thus, they may contain further nucleic acid sequences as well. Specifically, the polynucleotide of the present invention may encode e.g. polypeptides, including fusion polypeptides and selectable markers. Such polypeptides may comprise polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein. Selectable markers are known in the art and include in particular antibiotic resistance genes, selectable metabolic markers, e.g. an auxotrophy marker, and the like.

As used herein, the term "host cell" relates to a vertebrate cell. Preferably, the cell is a mammalian cell, more preferably, a human, mouse, rat, cat, dog, hamster, guinea pig, sheep, goat, pig, cattle, or horse cell. Still more preferably, the host cell is a primate cell. Most preferably, the host cell is a human cell. Preferably, the host cell is a heart muscle cell, more preferably a cardiomyocyte. More preferably, the host cell is a human heart muscle cell, more preferably a human cardiomyocyte. Moreover, a "host cell comprising the polynucleotide" of the present invention may also be a bacterial, yeast, or insect cell, preferably a bacterial cell of the genus Escherichia, more preferably an Escherichia coli cell, in particular in case the polynucleotide is or is comprised in a vector.

Advantageously, it was found in the work underlying the present invention that by introducing a miRNA of the present invention into a host cell, in particular a heart muscle cell, GRK2 expression can be reduced over a long period of time, mediating a maintenance of heart strength, improving calcium handling by the heart muscle cells, and/or maintaining responsiveness to catecholamines.

The definitions made above apply mutatis mutandis to the following. Additional definitions and explanations made further below also apply for all embodiments described in this specification mutatis mutandis.

The present invention further relates to a polyribonucleotide encoded by a polynucleotide according to the present invention; to a vector comprising the polynucleotide according to the present invention and/or encoding the polyribonucleotide according to the present invention.

The present invention also relates to a host cell comprising the polynucleotide according to the present invention, the polyribonucleotide according to the present invention, and/or the vector according to the present invention.

The present invention also relates to a polynucleotide according to the present invention, a polyribonucleotide according to the present invention, a vector according to the present invention, and/or a host cell according to the present invention for use in medicine; and to a polynucleotide according to the present invention, a polyribonucleotide according to the present invention, a vector according to the present invention, and/or a host cell according to the present invention for use in treatment and/or prevention of heart failure in a subject, preferably chronic heart failure, hypertrophic cardiomyopathy, dilatative cardiomyopathy, ischemic cardiomyopathy, and/or coronary heart disease.

The terms "treatment" and "treating", as used herein, refer to an amelioration of a disease or disorder referred to herein or the symptoms accompanied therewith to a significant extent. Said treating as used herein also includes an entire restoration of health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 20%, more preferably at least 50%, even more preferably at least 60%, still more preferably at least 70%, still more preferably at least 80%, most preferably at least 90% of the subjects of a given cohort or population. As will be understood by the skilled person, effectiveness of treatment is dependent on a variety of factors including, e.g. disease stage and severity. As will be understood, the term treatment includes measures preventing and/or delaying progression of a disease or disorder referred to herein.

The tem "preventing" refers to retaining health with respect to the diseases or disorders referred to herein for a certain period of time in a subject. It will be understood that said period of time is dependent on the amount of the drug compound which has been administered and individual factors of the subject discussed elsewhere in this specification. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification. As used herein, the term prevention, preferably, includes measures of gene therapy, as described elsewhere herein. Thus, the subject receiving preventive measures may be free of any symptoms of disease, but may be identified e.g. by gene analysis to have an increased risk of developing a disease or disorder referred to herein, or the subject receiving preventive measures may be identified by cardiac strain analysis via echocardiogram or MRI.

The term "heart failure" is known to the skilled person to relate to a pathological condition in which the heart of a subject is unable to maintain sufficient blood flow to meet the subject's body's needs. Preferably, heart failure is chronic heart failure or acute deterioration of pre-existing heart failure. Also preferably, heart failure is chronic heart failure, hypertrophic cardiomyopathy, dilatative cardiomyopathy, ischemic cardiomyopathy, and/or coronary heart disease. Symptoms and methods for diagnosing the aforesaid disorders and diseases are known to the skilled person, in particular from standard medical text books and, preferably, are those specified herein below.

The term "subject", as used herein, relates to a vertebrate, more preferably a mammal, even more preferably a livestock or companion animal, such as a chicken, a goose, a duck, a goat, a sheep, a cattle, a pig, a horse, a dog, a cat, a hamster, a rat, a mouse, an alpaca, a guinea pig, a rabbit, a hare, or a human. Still more preferably, the subject is a pig or a human, most preferably, the subject is a human. Preferably, the subject is suffering, preferably at the time of administration of a treatment as specified herein, from heart failure, more preferably was diagnosed to suffer from heart failure. Symptoms and diagnostic methods for diagnosing heart failure are known to the skilled person and include in particular fatigue, shortness of breath and other well-known symptoms; diagnostic tests for heart failure include in particular clinical chemistry, genetic testing, electrocardiogram, transthoracic and/or transesophageal echocardiography, cardiac MRI, cardiac CT, coronary angiography, right heart catheter, endomyocardial biopsy, chest X-ray, thoracic and/or abdominal ultrasound, spiroergometry, and the 6-min-walk-test.

Preferably, the use of the polynucleotide for treatment and/or prevention of heart failure comprises administration of the polynucleotide systemically and/or to the heart of a subject. Systemic administration may be intravenous administration; cardiac administration may be administration to at least one coronary arteria and/or coronary vein, intramyocardial administration, pericardial administration, and/or atrial and/or ventricular administration. Administration may preferably be performed by ultrasound targeted microbubble destruction. More preferably, said use comprises intravenous administration of from 10⁶ to 10¹⁵, preferably of from 10⁸ to 10¹³, viral particles comprising said polynucleotide to said subject; preferably, said viral particles are AAV viral particles comprising the polynucleotide as specified herein above. Also preferably, the subject further receives or is planned to further receive additional therapeutic measures for treating and/or preventing heart failure or one of its comorbidities, in particular at least one therapy selected from pharmacological therapy, implantable cardioverter defibrillator (ICD) therapy, cardiac resynchronization therapy (CRT), ventricular assist device (VAD) therapy, and heart transplantation. Preferably, the use comprises administration of the polynucleotide, polyribonucleotide, vector and/or host cell at most once a week, preferably at most once every two weeks, still more preferably at most every month, most preferably at most every 2 months.

The present invention also relates to a device comprising a polynucleotide according to the present invention, a polyribonucleotide according to the present invention, a vector according to the present invention, and/or a host cell according to the present invention.

The term "device", as used herein relates to a system of means comprising at least the means operatively linked to each other as to allow administration of the polynucleotide, polyribonucleotide, vector, and/or host cell of the present invention. Preferred means for administering polynucleotides, polyribonucleotides, vectors, and/or host cells are well known in the art. How to link the means in an operating manner will depend on the type of means included into the device and on the kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or composition of the invention. In another preferred embodiment, the device is an intravenous infusion (IV) equipment comprising the compound or composition. In another preferred embodiment, the device is an endoscopic device comprising the compound or medicament for flushing a site of administration, or further comprising a needle for topical application of the compound or composition, e.g. to a heart or blood vessel.

Preferably, the polynucleotide according to the present invention, the polyribonucleotide according to the present invention, the vector according to the present invention, and/or the host cell according to the present invention are provided as a pharmaceutical preparation, i.e., preferably, as a medicament.

The term "pharmaceutical preparation", as used herein, relates to a preparation comprising the compound or compounds of the present invention in a pharmaceutically acceptable form and a pharmaceutically acceptable carrier. The compounds of the present invention can be formulated as pharmaceutically acceptable salts. Acceptable salts comprise acetate, HCl, sulfate, chloride and the like. The pharmaceutical preparations are, preferably, administered topically or systemically. Suitable routes of administration conventionally used for drug administration are oral, intravenous, or parenteral administration as well as inhalation. Preferably, the pharmaceutical preparation of the present invention is administered via a parenteral route, preferably by intravenous, intraarterial, and/or intracardial administration. Moreover, the compounds can be administered in combination with other drugs either in a common pharmaceutical preparation or as separated pharmaceutical preparations wherein said separated pharmaceutical preparations may be provided in form of a kit of parts.

The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania.

The diluent(s) is/are preferably selected so as not to affect the biological activity of the polynucleotide, vector, or host cell and potential further pharmaceutically active ingredients. Examples of such diluents are distilled water, physiological saline, Ringer's solutions, dextrose solution, and Hank's solution. In addition, the pharmaceutical preparation or formulation may also include other carriers, adjuvants, or nontoxic, nontherapeutic, nonimmunogenic stabilizers and the like.

A therapeutically effective dose refers to an amount of the compounds to be used in a pharmaceutical preparation of the present invention which prevents, ameliorates or treats a condition referred to herein. Therapeutic efficacy and toxicity of compounds can be determined by standard pharmaceutical procedures in cell culture or in experimental animals, e.g., by determining the ED50 (the dose therapeutically effective in 50% of the population) and/or the LD50 (the dose lethal to 50% of the population). The dose ratio between therapeutic and toxic effects is the therapeutic index, and it can be expressed as the ratio, LD50/ED50.

The dosage regimen will be determined by the attending physician, preferably taking into account relevant clinical factors and, preferably, in accordance with any one of the methods described elsewhere herein. As is well known in the medical arts, a dosage for any one patient may depend upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 µg to 10000 µg; however, doses below or above this exemplary range are envisioned, especially considering the aforementioned factors. Depending on the subject and the mode of administration, the quantity of substance administration may vary over a wide range to provide from about 0.01 mg per kg body mass to about 1 mg per kg body mass, preferably. The pharmaceutical preparations and formulations referred to herein are administered at least once in order to treat or prevent a disease or condition recited in this specification.

The present invention also relates to a kit comprising a polynucleotide according to the present invention, a polyribonucleotide according to the present invention, a vector according to the present invention, and/or a host cell according to the present invention, comprised in a housing.

The term "kit", as used herein, refers to a collection of the aforementioned compounds, means or reagents which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to herein above. It is, preferably, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for administration and/or dosage instructions for carrying out the aforementioned methods using the kit of the present invention. Preferably, the kit further comprises at least one of a device according to the present invention and a diluent, preferably as specified herein above.

The present invention further relates to a method for treating heart failure in a subject, comprising
i) contacting said subject with a polynucleotide according to the present invention, a polyribonucleotide according to the present invention, a vector according to the present invention, and/or a host cell according to the present invention, and
ii) thereby treating heart failure.

The method of the present invention, preferably, is an in vivo method. Moreover, it may comprise steps in addition to those explicitly mentioned above. For example, further steps may relate, e.g., to providing a polynucleotide, a polyribonucleotide, a vector , and/or a host cell for step i), and/or further treatment steps in step b). Moreover, one or more of said steps may be performed by automated equipment.

In view of the above, the following embodiments are particularly envisaged:
Embodiment 1: A polynucleotide comprising and/or encoding a microRNA (miRNA) decreasing G protein-coupled receptor kinase 2 (GRK2) gene expression in a host cell.
Embodiment 2: The polynucleotide of embodiment 1, wherein said miRNA comprises, preferably consists of, a nucleic acid sequence (miRNA sequence) being the reverse complement of a GRK2 target nucleic acid sequence (GRK2 target sequence).
Embodiment 3: The polynucleotide of embodiment 1 or 2, wherein said GRK2 target sequence consists of at least 15, preferably of from 18 to 22, more preferably 21, contiguous nucleotides of a nucleic acid sequence at least 70% identical to a GRK2 encoding polynucleotide, preferably a GRK2 encoding mRNA.
Embodiment 4: The polynucleotide of any one of embodiments 1 to 3, wherein said GRK2 target sequence consists of at least 15, preferably of from 18 to 22, more preferably 21, contiguous nucleotides of a nucleic acid sequence of a GRK2 encoding polynucleotide, preferably of a GRK2 encoding mRNA.
Embodiment 5: The polynucleotide of any one of embodiments 1 to 4, wherein said GRK2 target sequence comprises, preferably consists of, the sequence of any one of SEQ ID NO:s:6 to 10.
Embodiment 6: The polynucleotide of any one of embodiments 2 to 5, wherein said polynucleotide comprises and/or encodes said miRNA sequence, a spacer sequence, and a passenger sequence, preferably in a 5' to 3' order, wherein said passenger sequence is the reverse complement of said miRNA sequence but lacks at least one nucleotide corresponding to a position of from 4 to 12 of said reverse complement of said miRNA sequence.
Embodiment 7: The polynucleotide of embodiment 6, wherein said passenger sequence lacks at least the nucleotides, preferably lacks the nucleotides, corresponding to positions 9 and 10 of said reverse complement of said miRNA sequence.
Embodiment 8: The polynucleotide of any one of embodiments 1 to 7, wherein said miRNA consists of a nucleic acid sequence selected from SEQ ID NOs:1 to 5 or a sequence at least 80% identical thereto; preferably consists of a nucleic acid sequence selected from SEQ ID NOs:1 to 5.
Embodiment 9: The polynucleotide of any one of embodiments 1 to 8, further comprising a nucleic acid sequence selected from SEQ ID NOs:11 to 15 or a sequence at least 80% identical thereto; preferably comprising a nucleic acid sequence selected from SEQ ID NOs:11 to 15.
Embodiment 10: The polynucleotide of any one of embodiments 1 to 9, wherein said polynucleotide comprises nucleic acid sequences of (i) SEQ ID NOs:1 and 11, (ii) SEQ ID NOs:2 and 12, (iii) SEQ ID NOs:3 and 13, (iv) SEQ ID NOs:4 and 14, (v) SEQ ID NOs:5 and 15, or (vi) any combination of (i) to (v).
Embodiment 11: The polynucleotide of any one of embodiments 1 to 10, wherein said polynucleotide comprises a nucleic acid sequence selected from SEQ ID NOs:16 to 20 or a sequence at least 80% identical thereto; preferably consists of a nucleic acid sequence selected from SEQ ID NOs:16 to 20.
Embodiment 12: The polynucleotide of any one of embodiments 1 to 11, wherein said polynucleotide further comprises a promoter causing expression of said polynucleotide in said host cell, preferably a myosin light chain promoter or active fragment thereof.
Embodiment 13: The polynucleotide of any one of embodiments 1 to 12, wherein said polynucleotide further comprises the sequence of any one of SEQ ID NOs:26 to 27..
Embodiment 14: The polynucleotide of any one of embodiments 1 to 13, wherein said polynucleotide comprises, preferably consists of, the nucleic acid sequence of any one of SEQ ID NOs:21 to 25, or a sequence at least 80% identical thereto, preferably comprises the nucleic acid sequence of any one of SEQ ID NOs: 21 to 25.
Embodiment 15: The polynucleotide of any one of embodiments 1 to 14, wherein said polynucleotide comprises or encodes at least one further miRNA.
Embodiment 16: The polynucleotide of any one of embodiments 1 to 15, wherein said polynucleotide comprises or encodes at least one further miRNA as specified in any one of embodiments 1 to 15.
Embodiment 17: The polynucleotide of any one of embodiments 1 to 16, wherein said polynucleotide comprises at least one packaging signal for a vector, preferably for an adeno-associated virus (AAV), more preferably for AAV9.
Embodiment 18: The polynucleotide of any one of embodiments 1 to 17, wherein said polynucleotide is comprised in a vector packaging construct, preferably a viral vector packaging construct, more preferably an adeno-associated virus (AAV) packaging construct, preferably an AAV9 packaging construct.
Embodiment 19: The polynucleotide of any one of embodiments 1 to 18, wherein said host cell is a vertebrate cell, preferably a mammalian cell, more preferably a human cell.
Embodiment 20: The polynucleotide of any one of embodiments 1 to 19, wherein said host cell is a heart muscle cell, preferably a cardiomyocyte.
Embodiment 21: A polyribonucleotide encoded by a polynucleotide according to any one of embodiments 1 to 20.
Embodiment 22: A vector comprising the polynucleotide according to any one of embodiments 1 to 20 and/or encoding the polyribonucleotide according to embodiment 21.
Embodiment 23: The vector according to embodiment 22, wherein said vector is an adeno-associated virus (AAV), preferably AAV9.
Embodiment 24: A host cell comprising the polynucleotide according to any one of embodiments 1 to 20, the polyribonucleotide according to embodiment 21, and/or the vector according to embodiment 22 or 23.
Embodiment 25: A polynucleotide according to any one of embodiments 1 to 20, a polyribonucleotide according to embodiment 21, a vector according to embodiment 22 or 23, and/or a host cell according to embodiment 24 for use in medicine.
Embodiment 26: A polynucleotide according to any one of embodiments 1 to 20, a polyribonucleotide according to embodiment 21, a vector according to embodiment 22 or 23, and/or a host cell according to embodiment 24 for use in treatment and/or prevention of heart failure in a subject, preferably chronic heart failure, hypertrophic cardiomyopathy, dilatative cardiomyopathy, ischemic cardiomyopathy, and/or coronary heart disease.
Embodiment 27: The polynucleotide, polyribonucleotide, vector, and/or host cell for use of embodiment 26, wherein said use comprises administration of said polynucleotide, polyribonucleotide, and/or host cell at most once a week, preferably at most once every two weeks, still more preferably at most every month, most preferably at most every 2 months.
Embodiment 28: A device comprising the polynucleotide according to any one of embodiments 1 to 20, a polyribonucleotide according to embodiment 21, a vector according to embodiment 22 or 23, and/or a host cell according to embodiment 24.
Embodiment 29: A kit comprising the polynucleotide according to any one of embodiments 1 to 20, a polyribonucleotide according to embodiment 21, a vector according to embodiment 22 or 23, and/or a host cell according to embodiment 24, comprised in a housing.
Embodiment 30: The kit of embodiment 29, further comprising at least one of a device according to embodiment 28 and a diluent.
Embodiment 31: A method for treating heart failure in a subject, comprising
   i) contacting said subject with the polynucleotide according to any one of embodiments 1 to 20, a polyribonucleotide according to embodiment 21, a vector according to embodiment 22 or 23, and/or a host cell according to embodiment 24, and
   ii) thereby treating heart failure.
Embodiment 32: The method of embodiment 31, wherein said subject is a mammal, preferably a human.
Embodiment 33: Use of a polynucleotide according to any one of embodiments 1 to 20, a polyribonucleotide according to embodiment 21, a vector according to embodiment 22 or 23, and/or a host cell according to embodiment 24 for manufacturing a medicament for the treatment and/or prevention of heart failure.

All references cited in this specification are herewith incorporated by reference with respect to their entire disclosure content and the disclosure content specifically mentioned in this specification.

### Figure Legends

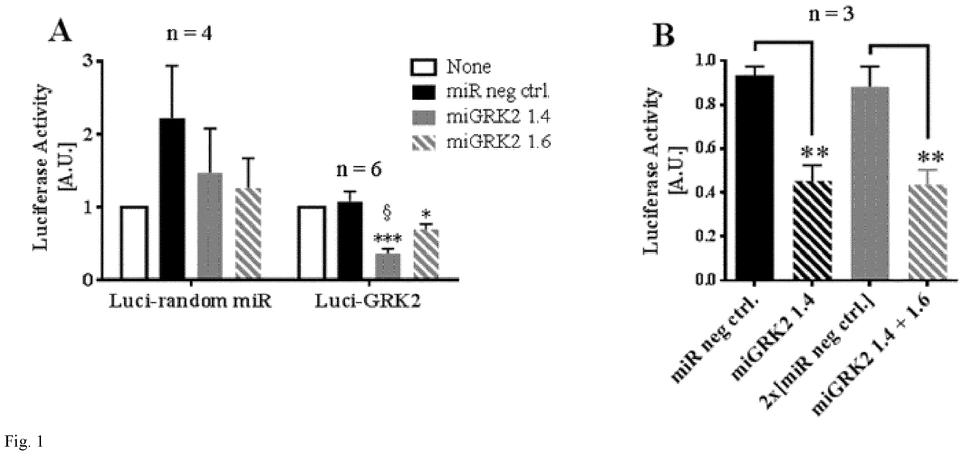

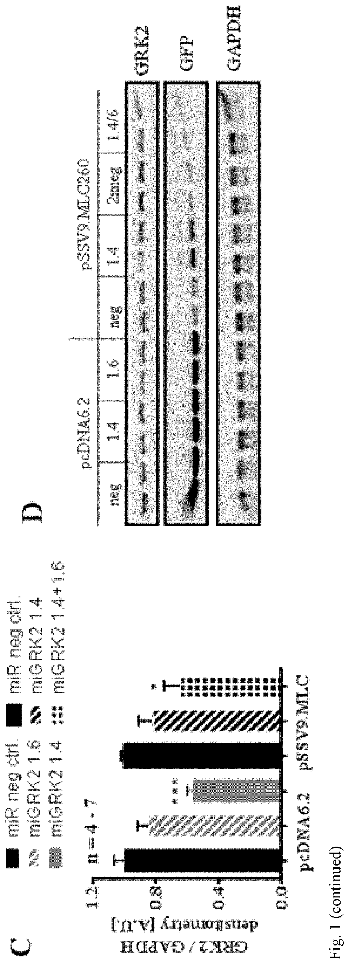

**Fig. 1****: miGRK2 induces significant knockdown of GRK2 in vitro. (A)** miGRK2 1.4 and miGRK2 1.6 induce a significant knockdown of GRK2 in vitro. HEK293 cells were co-transfected with plasmids encoding different miGRK2 sequences and a plasmid encoding firefly luciferase and mouse GRK2 (Luci-GRK2). A plasmid encoding random nucleotides was used as a control (Luci-random miR). **(B)** Combination of both miGRK2 sequences slightly improves knockdown efficiency in vitro. A dual-miRNA construct was cloned which was harboring both miGRK2 1.4 and 1.6 in two independent expression cassettes. HEK cells were co-transfected with Luciferase-GRK2 plasmid and luciferase activity was measured. **(C) & (D)** A combination of miGRK2 1.4 and 1.6 increases knockdown efficiency in H9c2 cells. GRK2 knockdown quantification and representative western blot of H9c2 cells transfected with plasmids encoding different miGRK2-plasmids. Statistics: One-Way ANOVA with Tukey's post-hoc testing. * P < 0.05, ** P < 0.01, *** P < 0.001, § P < 0.05 for comparison of miGRK2 1.4 vs. miGRK2 1.6. All data presented as mean ± SEM.

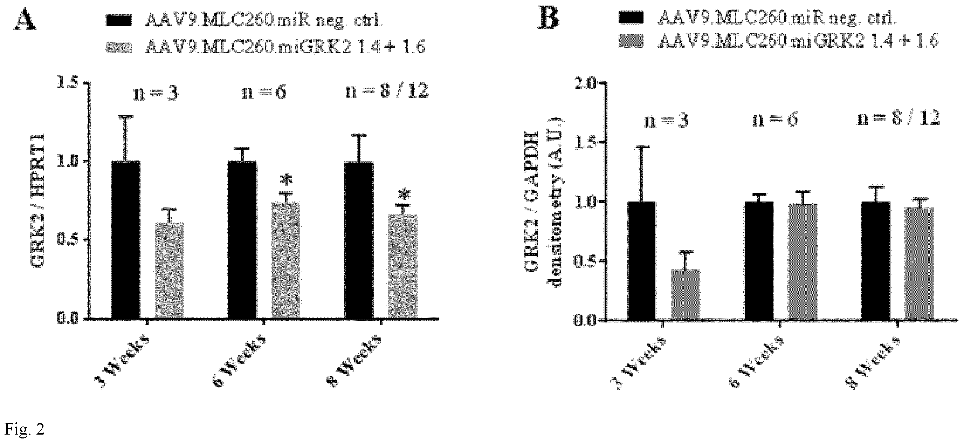

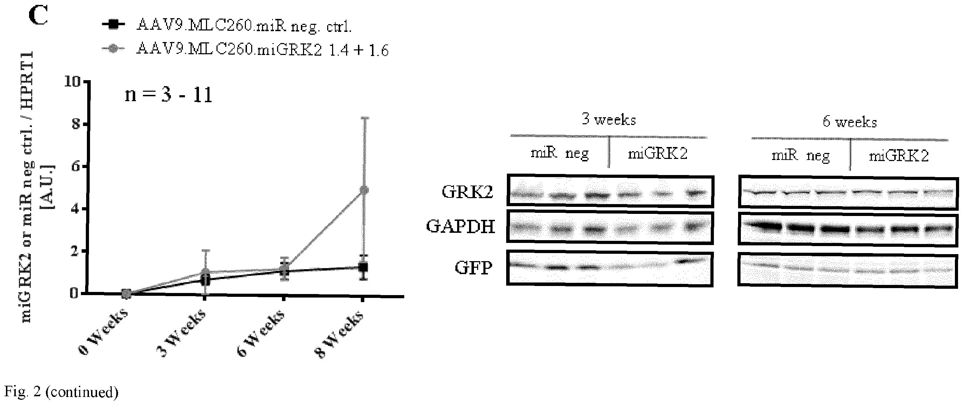

**Fig. 2****: AAV9.miGRK2 induces a significant and robust mRNA knockdown *in vivo.* (A)** Quantification of GRK2 mRNA from left-ventricular myocardium at 3, 6 and 8 weeks after AAV injection reveals significant and stable mRNA knockdown. 38 mice were tail-vein injected with 1x10¹² viral genomes (vg) of either pSSV9.MCL260.2x[mir neg ctrl.] or pSSV9.MLC260.miGRK2 1.4&1.6. 3, 6 and 8 weeks after injection, mice were sacrificed and organs were harvested to perform qPCR **(A)** and protein **(B)** analysis of GRK2 levels, respectively. **(C)** Quantification of miGRK2 and miR neg ctrl. reveals sustained expression of miGRK2 and miR neg ctrl. RNA from left ventricles were isolated and the level of mature miR neg ctrl. or miGRK2 1.4 was quantified as described in the methods section. Bottom: Representative western blot at respective time points. Statistics: Mann-Whitney-U test * P < 0.05. All data presented as mean ± SEM.

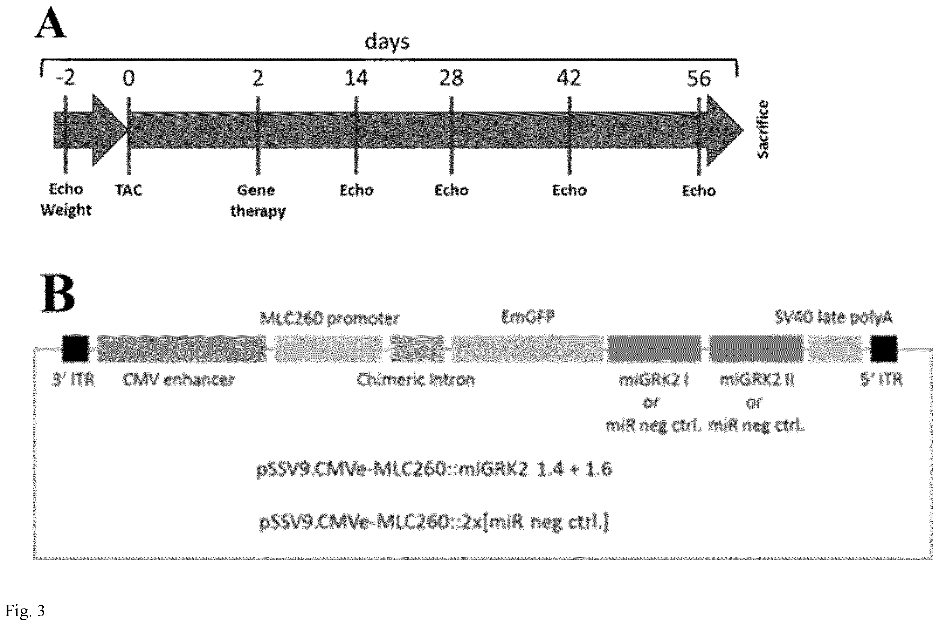

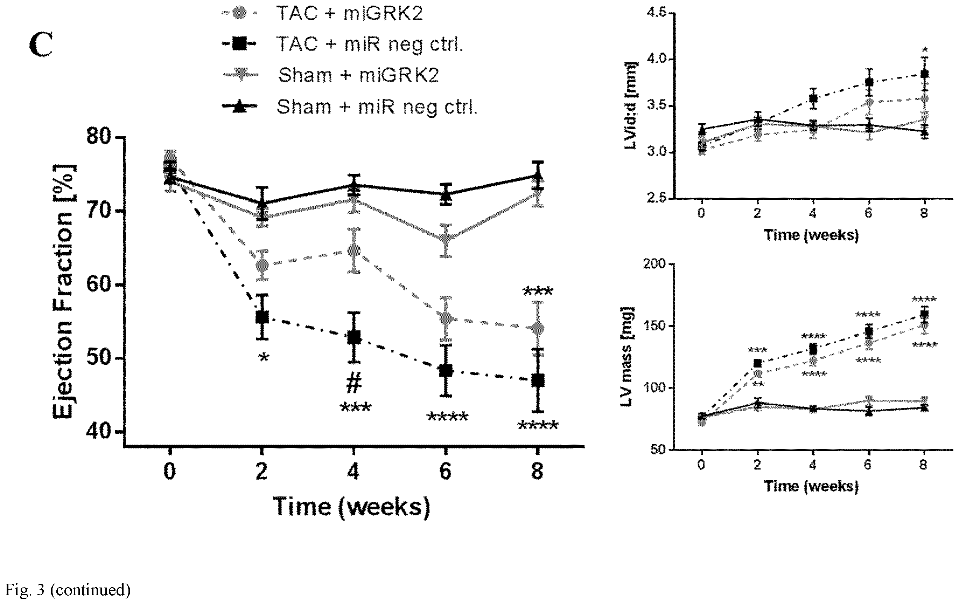

**Fig. 3****: AAV mediated expression of miGRK2 results in transient attenuation of HF progression. (A)** Study protocol used for in vivo testing of miGRK2 in pressure-overload induced heart failure. TAC: transverse aortic constriction. **(B)** Genetic organization of plasmids used for production of AAV9.miGRK2 or AAV9.miR neg ctrl. **(C)** Echocardiographic evaluation of systolic cardiac function and left ventricular geometry reveals a delayed cardiac remodeling and significantly attenuated heart failure progression four weeks following virus injection. (LVid;d): diastolic left ventricular diameter, (LV mass): calculated left ventricle mass. All data presented as mean ± SEM. Statistic: Two-ANOVA with Tukey's post-hoc testing. Ejection Fraction: * P < 0.05 for TAC vs. Sham + miR neg ctrl; *** P < 0.001 for TAC vs. Sham miR neg ctrl. or TAC vs. Sham + miGRK2; **** P < 0.0001 for TAC vs. Sham + miR neg ctrl.; # P < 0.05 TAC + miR neg ctrl. vs. miGRK2; LVid;d: * P < 0.05 for Sham vs. TAC + miR neg ctrl.; Lvmass [mg]: ** P < 0.01 for TAC vs. Sham + miGRK2; *** P < 0.001 for TAC vs. Sham + miR neg ctrl.; *** P < 0.0001 for TAC vs Sham + miR neg ctrl (top) or + miGRK2 (bottom); Sham + miR neg ctrl. n = 8; Sham + miGRK2 n = 12; TAC + miR neg ctrl. n = 25; TAC + miGRK2 n = 22

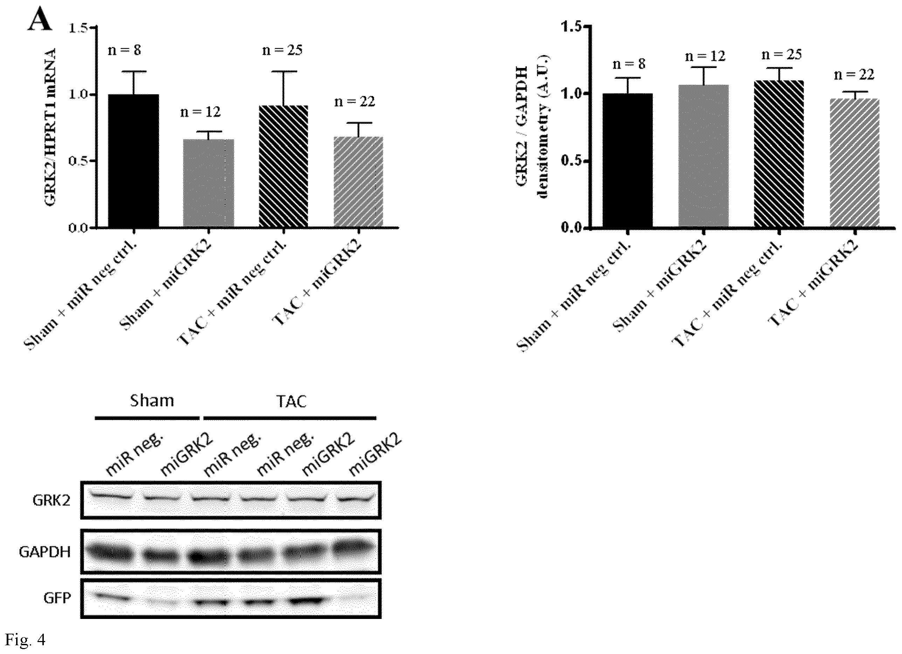

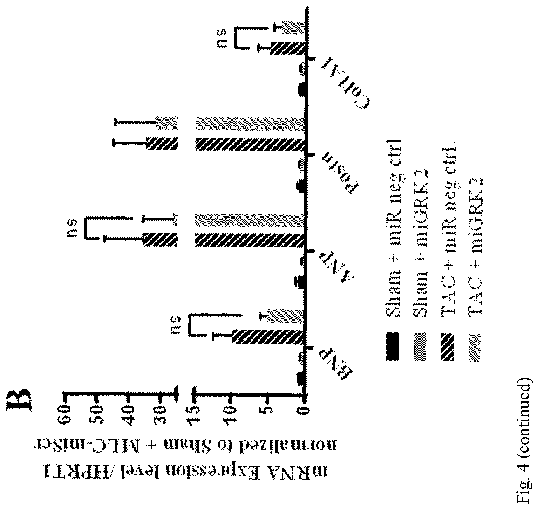

**Fig. 4****: Treatment with miGRK2 induces a robust downregulation of GRK2 on mRNA level. (A)** Quantification of GRK2 mRNA (top) and protein (bottom) expression with representative western blot (bottom) **(B)** mRNA expression of marker genes for HF and cardiac remodeling. BNP: brain natriuretic peptide, ANP: atrial natriuretic peptide, Postn1: Periostin 1, Col1A1: Collagen 1. All data presented as mean ± SEM. Statistics: One-Way ANOVA with Tukey's post-hoc testing. ns: P > 0.05

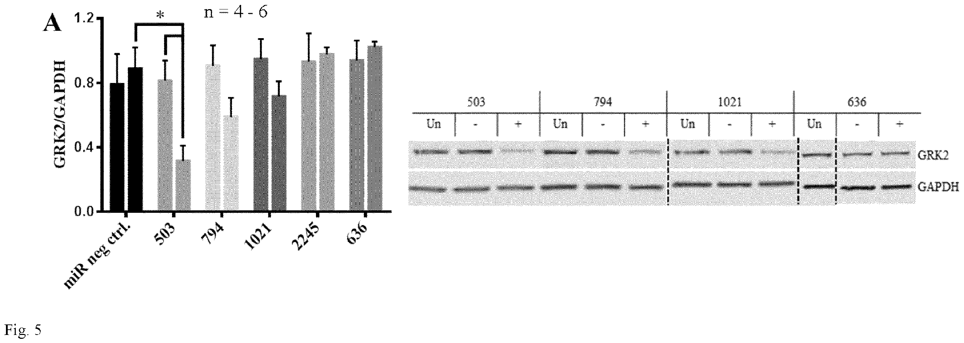

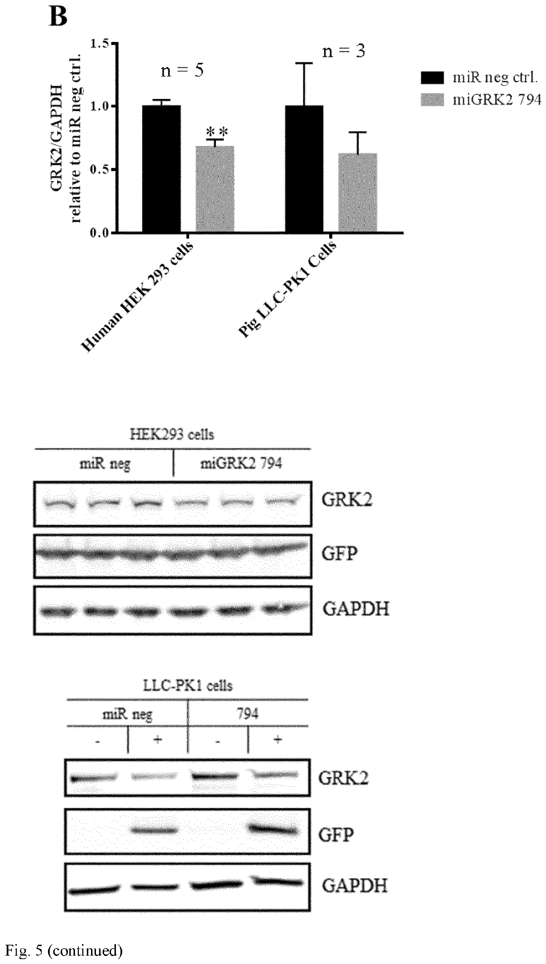

**Fig. 5****: Human- and pig-specific sequences show different knockdown efficiencies in vitro. (A)** HEK293 cells transfected with different miGRK2-encoding plasmids show significantly decreased levels of GRK2 when using miGRK2 503 and 794. HEK293 were transfected with different miRNA-encoding constructs, a sample of transfected cells was taken (unsorted; Un) and cells were sorted by FACS/Sorting of GFP-negative (left bar) and GFP-positive (right bar) cells. GRK2 and GAPDH were analyzed and quantified using Immunoblot (right panel). Statistics: Two-Way ANOVA with Tukey's post-hoc testing. * P < 0.05. **(B)** miGRK2 794 induces a significant knockdown of GRK2 in HEK293 cells and in LLC-PK1 cells. Human HEK293 cells and porcine LLC-PK1 cells were transfected with plasmids encoding miR neg ctrl. or miGRK2 793, proteins were isolated and immunoblotted for GRK2, GFP and GAPDH (right panel) and quantified (left panel). All data presented as mean ± SEM. Statistics: Mann-Whitney-U test; ** P < 0.01

The following Examples shall merely illustrate the invention. They shall not be construed, whatsoever, to limit the scope of the invention.

### 1 Design and Cloning of miRNA sequences

If not otherwise stated, standard chemicals and reagents were purchased from Sigma-Aldrich, St. Louis, MO, USA. Standard consumables were purchased from Greiner, Frickenhausen, Germany.

In order to establish a vector-based system capable of knocking down GRK2 *in vitro* and *in vivo,* a set of siRNA/miRNA sequences were designed and cloned into a CMV-driven plasmid. Design of miRNAs was conducted as described earlier (Chung et al. 2006. Nucleic Acids Res. 34, e53.) and was carried out using the BLOCK-iT Pol II miR RNAi Expression Vector Kit (ThermoFisher Scientific, Carlsbad, CA, USA) according to the manufacturer's recommendations. The online algorithm was used to generate a list of potential miRNA sequences based on the mRNA sequence NM_001290818.1 (*Mus musculus* adrenergic receptor kinase, beta 1 (Adrbk1)). In addition, a second set of sequences was generated by providing the tool with the mRNA sequence NM_012776.1 (*Rattus norvegicus* adrenergic, beta, receptor kinase 1 (Adrbk1)). By comparing the two lists, a set of sequences were identified to potentially target GRK2 in both mice and rat tissues. The sequences were cloned into the expression vectors provided with the kit. The resulting vectors pcDNA6.2-EmGFP-miRNA harboring different miRNA (miGRK2) sequences or a negative control sequence (miR neg ctrl.) were the basis for subsequent cloning steps and for first *in vitro* tests.

### 1.1 Cloning of Adeno-associated viral plasmids

In general, *E. coli* NEB 5α, which was used for all following cloning steps unless differently stated, and all DNA-modifying enzymes were purchased from New England Biolabs, Ipswich, MA, USA. Plasmids from *E. coli* were isolated using the GeneElute Mini Kit (Sigma-Aldrich), DNA from gel fragments was retrieved using the QIAquick Gel Extraction Kit (Qiagen, Hilden, Germany) and DNA from solutions was purified using the QIAquick PCR Purification Kit according to each manufacturers' protocols. For long-term storage and large-scale production of viral plasmids derived from either pDS or pSSV9 backbones, *E. coli* SURE2 (Agilent Technologies, Willoughby, Australia) was used because of its superior ability to keep inverted terminal repeats (ITRs) intact. *E. coli* were stored in 25 % glycerol at -80°C for long-term storage. Large-scale plasmid production was carried out using the Plasmid Giga Kit (Qiagen).

First, the shuttle vector pDS-CMVₑₙₕ-MLC260 was digested with HpaI/SpeI/CIP, separated on a 1.2 % Agarose gel and the 4,055 bp fragment was gel-extracted as described above. Similarly, the plasmids generated under 1 were digested with SpeI/FspI, the 2,040 bp fragment was gel-extracted and cloned into the linearized backbone. The resulting plasmid was termed pDS-CMVₑₙₕ-MLC260-EGFP-miRNA, with miRNA being either miGRK2 1.4, miGRK2 1.6 or miR neg ctrl..

In order to generate a vector for production of Adeno-associated viruses (AAV) with an easily exchangeable promoter element, a synthetic fragment of the MLC260 promoter flanked by multiple-cloning sites was synthesized (^{MCS}MLC260^{MCS}, GeneArt, Thermo Fisher Scientific). The enhancer element of the cytomegalovirus (CMVₑₙₕ) was amplified from the plasmid pDS-CMVₑₙₕ-MLC260-EGFP using the primers 5'-ATA TAT TAG ATC TGA ATT CAC GCG TCG GCG GC-3' (SEQ ID NO:34) and 5'-ATA TAT TGT CGA CCA TGG GGC GGA GTT GTT ACG-3' (SEQ ID NO:35) and Phusion polymerase according to the manufacturer's recommendation. The PCR reaction was purified and the resulting fragment flanked by digestible overhangs was cut with BglII/SalI and cloned into pEGFP-N1 (Takara Bio Europe, Paris, France) linearized with BglII/SalI/CIP. The synthetic ^{MCS}MLC260^{MCS} fragment was amplified using the primers 5'-ATA TAT TGT CGA CAA GCT TCC TGC AGG CGC GC-3' (SEQ ID NO:36) and 5'-ATA TAT TGG ATC CCT GTG GAG AGA AAG GCA AAG TGG-3' (SEQ ID NO:37), digested with SalI/BamHI and cloned into the aforementioned pEGFP-N1::CMVₑₙₕ, thereby generating a plasmid termed pEGFP-N1::CMVₑₙₕ-^{MCS}MLC260^{MCS}.

This plasmid was digested with EcoRI/BamHI and the resulting 1,022 bp fragment comprising the CMVₑₙₕ-^{MCS}MLC260^{MCS} was gel-purified as described above. The vector pDS-CMVₑₙₕ-MLC260-EGFP, which served as a shuttle vector in this step was linearized with EcoRI/BamHI/CIP and the resulting 4,864 bp fragment was gel-extracted and used for a ligation reaction. The resulting plasmid pDS-CMVₑₙₕ-^{MCS}MLC260^{MCS}-EGFP as well as the original plasmids pcDNA6.2-EmGFP-miRNA generated in the first step were digested with AgeI/BsrGI. The resulting 5,164 bp and 939 bp fragments, respectively, were gel-extracted and ligated, resulting in a vector termed pDS-CMVₑₙₕ-^{MCS}MLC260^{MCS}-EGFP-miRNA.

A Phusion PCR was run on this vector using the primers 5'-GAG CTC GCC CGA CAT TGA TTA TTG-3' (SEQ ID NO:38) and 5'-GAT TAC CGG TAG ATC TGG GCC ATT TGT T-3' (SEQ ID NO:39). The PCR product was purified as described above. The purified PCR product as well as the backbone pSSV9-CMV was digested with AgeI/SpeI. Both digests were separated on an Agarose gel and the 3,264 bp and 1,899 bp band, respectively, were extracted. The subsequent ligation obtained the single-stranded plasmid pSSV9-CMVₑₙₕ-^{MCS}MLC260^{MCS}-EGFP::*miRNA,* with *miRNA* being either miR neg ctrl, miGRK2 1.4 or miGRK2 1.6.

### 1.2 Generation of double-miRNA plasmids

Since the target sites of the two sequences reside in two distinct regions within GRK2 mRNA, we speculated that a combination of both sequences might enhance knockdown efficiency. Therefore, the two artificial miRNA cassettes comprising miGRK2 1.4 and 1.6 were cloned *in tandem* as recommended by the manufacturer. In brief, pSSV9-CMVₑₙₕ-^{MCS}MLC260^{MCS}-EGFP::miGRK2 1.4 or pSSV9-CMVₑₙₕ-^{MCS}MLC260^{MCS}-EGFP miR neg. ctrl. were digested with BglII/XhoI and CIP. pcDNA6.2-EmGFP::miGRK2 1.6 or miR neg. ctrl. were digested with XhoI/BamHI and the resulting 151 bp band was gel-extracted and ligated into CMVₑₙₕ-^{MCS}MLC260^{MCS}-EGFP::miGRK2 1.4 or CMVₑₙₕ-^{MCS}MLC260^{MCS}-EGFP::miR neg. ctrl., respectively.

### 2 Testing miRNA sequences in vitro

### 2.1 GRK2 Luciferase assay

### 2.1.1 Cloning

To test the different miGRK2 sequences an *in vitro* Luciferase assay was established. pCMV-luc-miR21 (P), encoding firefly luciferase and a miR21 target site, and pCMV-luc-random, in which the miR21-target site is replaced by non-sense nucleotides, were a gift from Bryan Cullen (Addgene plasmid # 20382 ; internet: n2t.net/addgene:20382; RRID:Addgene_20382) (Zeng, Y., and Cullen, B.R. 2003. RNA 9, 112-123). The plasmid pCMV-luc-miR21 was digested with XhoI, inactivated and digested for exactly 5 min with NcoI-HF. The resulting 5,200 bp fragment was immediately gel-extracted, treated with calf intestinal alkaline phosphatase (CIP) and DNA-purified. In order to obtain a miGRK2 target site, total RNA from mouse hearts was isolated and reversely transcribed.

### 2.1.2 RNA isolation from cells or tissues

In brief, 10 - 15 mg left ventricular tissue was homogenized using ceramic beads in a Precellys 24 bead mill homogenizer (Bertin Technologies SAS, Montigny-le-Bretonneux, France) in 1 ml Trizol (Thermo Fisher Scientific, Waltham, MA, USA) for 30 sec., 5,500 rpm. The homogenate was incubated for 5 min on ice and the homogenization was repeated once. The following RNA isolation was done according to the manufacturer's recommendation. For generation of cDNA purified RNA was transcribed using iScript cDNA Synthesis Kit (BioRad Laboratories GmbH, Munich, Germany) according to the manufacturer's recommendation. In brief, 1 µg of RNA was used in a 20 µl reaction volume. cDNA was diluted to 0.5 ng/µl and stored at -20°C for subsequent qPCR analysis or cloning steps.

### 2.1.3 qPCR

7.5 µl iQ SYBR green supermix (BioRad Laboratories) were used in a reaction containing primers at a final concentration of 101 nM and 3.25 ng cDNA. qPCR was run on a CFX qPCR cycler platform according to the manufacturer's recommendation.

### 2.1.4 Amplification of GRK2

GRK2 was amplified from 100 ng of mouse cDNA with Phusion High Fidelity Polymerase and the primers 5'-CGA CCC ATG GCC GAC CTG GAG GC-3' (SEQ ID NO:40) and 5'-CAG CCT CGA GTC AGA GGC CGT TGG CAC-3' (SEQ ID NO:41). The resulting 2,100 bp band was gel-extracted and blunt-cloned using the Zero Blunt PCR Cloning Kit (Thermo Fisher) according to the manufacturer's protocol. The resulting vector was transformed and amplified in *E. coli* DH5α, isolated and digested with NcoI/XhoI. The resulting 2,100 bp band was gel-extracted and ligated into pCMV-luc generated above with T4 ligase in a 1:3 ratio.

### 2.1.5 Luciferase activity assay

Luciferase activity was measured using the Luciferase Assay System (Promega, Mannheim, Germany) according to the manufacturer's recommendation. In brief, 1.5x10⁵ HEK 293T cells (ATCC, Manassas, VA, USA) were seeded on a 12-well plate for 24 h and transfected in triplicates with 600 ng of miRNA plasmid and luciferase reporter plasmid in a 1:1 molar ratio diluted in 150 mM NaCl and 1 mM Polyethylenimine (Polysciences, Warrington, PA, USA). 48 h after transfection, cells were lysed using 1 x RLB buffer and one additional freeze-thaw step was included to ensure complete cell lysis. 20 µl of supernatant were mixed with 100 µl luciferase substrate (Promega) in a 96 well plate in triplicates and the luciferase activity was measured on a LuminoSkan plate reader (ThermoFisher Scientific). Signals were normalized to the protein concentration of lysate measured with the DC Protein Assay Kit (BioRad Laboratories) according to the manufacturer's recommendation.

The data indicate that two of the tested sequences denoted miGRK2 1.4 and miGRK2 1.6 induce a significant knockdown of mouse GRK2 in the described luciferase *in vitro* assay (Fig. 1 B). The sequences of these miRNAs were: miGRK2 1.4: 5'-ATT CGA TGC ACA CTG AAG TCA-3' (SEQ ID NO:42); miGRK2 1.6: 5'-AGA AGT CAC AGG CTA GGC CCA-3' (SEQ ID NO:43). The sequence of the non-sense miRNA that served as negative control (miR neg ctrl.) was 5'-AAA TGT ACT GCG CGT GGA GAC-3' (SEQ ID NO:44).

We next tested whether a combination of both sequences offers benefits over the use of single sequences. Therefore, a luciferase assay was run with the double-miGRK2 plasmid as indicated. In fact, this plasmid induced a slightly higher knockdown of GRK2 compared to single sequences (Fig. 1 D). To test how these plasmids perform in other murine cell, H9c2 cells were obtained and tested.

### 2.2 Testing in a rat cell line

To further test the generated sequences *in vitro,* the immortalized cardiac rat cell line H9c2 was used. H9c2 cells were obtained from ATCC and kept in DMEM (Sigma-Aldrich, #D5671, with 4500 mg/L glucose) supplemented with 10 % FCS (Sigma-Aldrich) and 1 x Penicillin-Streptomycin (Thermo Fisher Scientific) with medium renewal every second day. In brief, 2x10⁵ cells were seeded in a 6 well plate and transfected with 3.75 µl Lipofectamine 3000 (Thermo Fisher Scientific) and 1 µg plasmid DNA according to the manufacturer's recommendations. The cells were incubated for 24 - 72 h without changing the medium at 37°C, 5 %, followed by cell lysis and protein isolation.

### 2.2.1 Protein isolation and immunoblot

1 - 2x10⁶ cells were lysed in 50 µl SDS buffer (KCl 2.67 mM, KH₂PO₄ 1.47 mM, NaCl 137.93 mM, Na₂HPO₄-7H₂O 8.06 mM, EDTA 1 mM, EGTA 1 mM, SDS 1 % (v/v)), supplemented with cOmplete, EDTA-free Protease Inhibitor Cocktail (Roche, Mannheim, Germany) and Phosphatase Inhibitor Cocktail 2&3 (Sigma-Aldrich)) and incubated for 30 min on ice. 10 - 15 mg tissue was homogenized using 300 µl SDS buffer and ceramic beads in a Precellys 24 bead mill homogenizer for 2 x 30 sec. at 6,000 rpm. After each homogenization step, protein lysates were incubated on ice for 5 min. Lysates were centrifuged for 15 min., 18,000 xg, 4°C to remove debris. Protein concentration of supernatant was determined using DC Protein Assay Kit (BioRad Laboratories) in a 96-well plate format with a BSA standard curve in duplicates and triplicates, respectively. Typically 50µg denatured protein was separated on a 4 - 20 % gradient Tris-Gylcine gel (ThermoFisher Scientific) and transferred to a PVDF membrane (Millipore, Dundee, UK) in a Trans-Blot Semidry blotting chamber (BioRad Laboratories). After blocking in iBlock solution for 1h the following antibodies were used diluted in iBlock solution: GRK2 (Santa Cruz Biotechnology, Heidelberg, Germany, #sc-562), GFP (Cell Signaling Technologies, Danvers, MA, USA; # 2956), PLN (Thermo Fisher Scientific, # MA3-922), Thr17-pPLN (Badrilla, Leeds, UK #A010-13AP), GAPDH (Millipore/Merck, Darmstadt, Germany #MAB374), Secondary antibodies were goat-a rabbit-HRP (SantaCruz #sc-2004), goat-a mouse-HRP (SantaCruz #sc-2302), Alexa Fluor 680 α-mouse (Thermo Fisher Scientific, #A-21109), DyLight 800 4X PEG Conjugate α-rabbit (Cell Signaling Technologies, #5151P). For HRP-coupled antibodies, SignalFire ECL Detection Kit was used (Cell Signaling Technologies).

H9c2 cells were transfected with plasmids encoding either miGRK2 1.4, 1.6 or a combination of both compared to plasmids encoding a control sequence under control of a strong ubiquitous CMV promoter or a cardio-specific MLC260 promoter (Fig. 1C and D). In order to control for the expected increase in miRNA processing by introducing a second miRNA, a plasmid containing two copies of miR neg ctrl. were used. Analysis of protein levels indicate that miGRK2 1.4 induces a strong reduction in GRK2 protein level, whereas miGRK2 1.6 leads to an only moderate reduction in GRK2 level. Surprisingly a construct combining both miGRK2 1.4 and 1.6 in a MLC260-driven plasmid had an even stronger effect compared to miGRK2 1.4 alone. GFP levels were comparable, indicating equal DNA amounts used for transfection of cells. Since the difference observed was so pronounced the plasmid comprising both miGRK2 1.4 and 1.6 termed pSSV9.MLC260.miGRK2 1.4&1.6 was used for all subsequent steps. The arrangement of genetic elements in this vector is illustrated in Figure 3B.

### 3 Testing of miGRK2 sequences in vivo

### 3.1 Production of Adeno-associated Viruses

Adeno-Associated Virus (AAV) vectors were produced in a double-transfection approach as described before (Müller et al. 2006. Cardiovasc. Res. 70, 70-78.). In brief, HEK 293T were transfected with the vector plasmids pSSV9.MLC260.miGRK2 1.4&1.6 and pSSV9.MLC260.2x[miR neg ctrl.] and pDP9 providing the cap sequences for AAV-9 as well as adenoviral helper sequences. Cells and supernatants containing viral particles were harvested after 48h, purified by Iodixanol step gradient centrifugation, and quantified using real-time PCR.

### 3.2 Testing AAVs in vivo

The adeno-associated viruses were tested in male, healthy, 8-weeks old C57BL/6 mice. Mice were tail-vain injected with 1x10¹² vg of AAV9.MLC260.miGRK2 1.4&1.6 or AAV9.MLC260.2x[miR neg ctrl.] and sacrificed after 3, 6 and 8 weeks. Cardiac tissue was harvested, lysed and proteins and RNA were isolated as described before. The proteins were immunoblotted for GRK2, GAPDH and GFP (Fig. 2 B). RNA from cardiac tissues was isolated, reversely transcribed and tested for the expression of GRK2 and HPRT1 as described above (Fig. 2 A).

At all analyzed time points, AAV9.miGRK2 induced a significant reduction of GRK2 mRNA (Fig. 2A and B), indicating that miGRK2 induces a sustained knockdown of GRK2 *in vivo.* We next determined the level of mature miGRK2 or miR neg. ctrl. in animals treated with AAV9.miGRK2 or AAV9.miR neg. ctrl., respectively (Fig. 2 C).

### 3.2.1 Determination of miRNA expression

Detection of miRNAs was performed as described earlier (Chen 2005. Nucleic Acids Research 33, e179-e179.) with minor modifications. RNA was isolated as described above, and 3 µg of RNA were digested with RQ1 RNase-free DNase (Promega) according to the manufacturer's recommendation. Stem-Loop primer for mature miRNAs were designed and refolded as described before (Kramer et al. 2011. Current protocols in molecular biology CHAPTER, 10). Following primers for reverse transcription of miRNAs were used:

| | |
|---|---|
| miGRK2 1.4 | 5'-GTC GTA TCC AGT GCA GGG TCC GAG GTA TTC GCA CTG GAT ACG ACT GAC TT-3' (SEQ ID NO:45) |
| miR neg ctrl. | 5'-GTC GTA TCC AGT GCA GGG TCC GAG GTA TTC GCA CTG GAT ACG ACG TCT CC-3' (SEQ ID NO:46) |

1.2 µg of digested RNA was reversely transcribed using the High-Capacity cDNA Reverse Transcription Kit (Thermo Fisher Scientific) according to the manufacturer's recommendations, supplemented with a mix of each 200 nM refolded stem loop primers listed above. To control the reaction, 0.6 µg RNA was used in a setup without Reverse Transcriptase. Resulting cDNA was diluted 1:65 in H₂O_{DEPC}.

Real Time PCR was performed using SYBR Green Reaction Mix (Bio-Rad Laboratories) in a 96-well plate setup and run on a CFX qPCR cycler (BioRad). The following table lists all primers used:

| | **Sequence** | **Species specificity** | **SEQ ID NO:** |
|---|---|---|---|
| Standard Rev | 5'-CCA GTG CAG GGT CCG AGG TA-3' | - | 47 |
| miR neg ctrl. Fw | 5'-CTC GCA AAT GTA CTG CGC GT-3' | - | 48 |
| miGRK2 1.4 Fw | 5'-CGC GGC ATT CGA TGC ACA-3' | - | 49 |
| *Grk2* Fw | 5'-CCC TCT CAC CAT CTC TGA GC-3' | Mouse, rat | 50 |
| *Grk2* Rev | 5'-CGG TTG GGG AAC AAG TAG AA-3' | | 51 |
| *Hprt1* Fw | 5'-GAG GAG TCC TGT TGA TGT TGC CAG-3' | Mouse | 52 |
| *Hprt1* Rev | 5'-GGC TGG CCT ATA GGC TCA TAG TGC-3' | | 53 |
| *Anf* Fw | 5'-TGC CGG TAG AAG ATG AGG TC-3' | Mouse, rat | 54 |
| *Anf* Rev | 5'-TGC TTT TCA AGA GGG CAG AT-3' | | 55 |
| *Bnp* Fw | 5'-CTG AAG GTG CTG TCC CAG AT-3' | Mouse, rat | 56 |
| *Bnp* Rev | 5'-CCT TGG TCC TTC AAG AGC TG-3' | | 57 |
| *β-Mhc* Fw | 5'-GCC AAC ACC AAC CTG TCC AAG TTC-3' | Mouse, rat | 58 |
| *β-Mhc* Rev | 5'-TGC AAA GGC TCC AGG TCT GAG GGC-3' | | 59 |
| *Col-1a* Fw | 5'-GTG TTC CCT ACT CAG CCG TC-3' | Mouse | 60 |
| *Col-1a* Rev | 5'-ACT CGA ACG GGA ATC CAT CG-3' | | 61 |
| *Postn* Fw | 5'-ACT TCA GCT CCT GTA AGA ACT G-3' | Mouse | 62 |
| *Postn* Rev | 5'-AGG GCA GCA TTC ATA TAG CAC A-3' | | 63 |

Correct amplification of miRNAs was checked by electrophoresis and cloning of PCR products into Zero Blunt PCR Cloning vector, followed by sequencing of the mature miRNAs using standard sequencing primers.

Here, both mature miRNAs were stably expressed at all analyzed time points (Figure 2 C). The data obtained from this measurement even indicate a constant increase of expression from 3 to 8 weeks.

### 4 Therapeutic application in cardiac disease

### 4.1.1 TAC Operation

We speculated that a knockdown of GRK2 might have beneficial effects in heart failure. As heart failure model, the previously described Transverse aortic constriction (TAC) model was chosen (Rockman et al., 1991, Proc. Natl. Acad. Sci. 88: 8277-8281). 48 8 weeks old C57BL/6 mice were weighted prior to the operation. TAC operations were performed on day 0 using 26-gauge blunt needle effectively reducing the diameter of the transverse aorta to approximately 0.46 mm (Rockman et al., 1994, Proc. Natl. Acad. Sci. 91(7): 2694-2698). Cardiac function was assessed by echocardiography (Vevo 2100 VisualSonics) before TAC operation (day -2), before virus infusion (day 2), and at 2, 4, 6 and 8 weeks follow up (Fig. 3 A). Two days after TAC operation, animals were randomized to systemic injection of AAV9.miR neg ctrl. or AAV9.miGRK2 virus. Follow up assessments were performed on day 55 and the animals were sacrificed 3 days later. In addition, 18 mice served as control animals which underwent sham operation.

### 4.1.2 Cardiac function measurements

Echocardiography was performed using Vevo 2100, VisualSonics. The mice were shaved and held in prone position. Warm ultrasound coupling gel (37°C) was placed on the shaved chest area, and the MS400 transducer was positioned to obtain 2D B-mode parasternal long and short axis views and M-mode short axis view. The ejection fraction (EF), fractional shortening (FS), left ventricular internal diameter (LVId;d) and heart rate (HR) were calculated from 6 consecutive heartbeats in the M-mode using LV trace function.

### 4.1.3 miGRK2 therapy improves cardiac function

2 weeks after surgery mice started to show a decline in cardiac function with reduced Ejection Fraction (EF) and significantly increased left ventricle mass (Figure 3 C). 4 weeks after surgery, mice treated with AAV9.miGRK2, however, showed a significantly improved EF and a normalized LV inner diastolic diameter (LVId;d, Fig. 3C top right panel) compared to mice which received control vectors. 6 and 8 weeks after surgery, cardiac output remained slightly higher in mice treated with AAV9.miGRK2 when compared to AAV9.miR neg. ctrl.-treated mice. However, progression of hypertrophy was not delayed or blocked in mice receiving AAV9.miGRK2 (Fig. 3C bottom right panel). At the end of the study, mice were sacrificed, organs were explanted and GRK2 mRNA and protein levels were determined as described above (Fig. 3µD). AAV9.miGRK2 treatment resulted in 35 % reduction of GRK2 mRNA. Markers for heart failure and fibrosis were significantly elevated in TAC-operated mice, but treatment with AAV9.miGRK2 did not result in a significant improvement of molecular markers.

### 5 Human-specific miGRK2 sequences

In a first set of experiments it was shown that miGRK2 sequences are capable of knocking down GRK2 *in vitro* and *in vivo* and that AAV-mediated knockdown of GRK2 offers benefits in a mouse model of heart failure. Next, we examined sequences capable of knocking down the human version of GRK2.

### 5.1 Design, cloning and in vitro-testing of human-specific miGRK2

The BLOCK-iT Pol II miR RNAi Design tool was provided with the mRNA sequences NM_001619.3 (*Homo sapiens* adrenergic, beta, receptor kinase 1 (ADRBK1)). The screen identified a list of promising sequences which were used to clone vectors as described above. In addition, a second set of sequences was created by feeding the tool with the mRNA sequence NM_001244878.1 (*Sus scrofa* adrenergic, beta, receptor kinase 1 (ADRBK1)). A sequence both active against human and porcine GRK2 offers the advantage of potential pretest in a large animal model prior to therapeutic use in men. By comparing the two lists, two sequences were identified to potentially target GRK2 in both human and porcine tissue. In total, 6 miRNA sequences were generated, cloned and tested *in vitro.* For testing, the human embryonic kidney cell line HEK293 and a porcine kidney cell line (Hull et al. 1976. In vitro 12, 670-677.) were purchased from ATCC. 1.185x10⁶ cells were seeded in a 10 cm dish in culture medium (HEK 293T: DMEM, 10 % (v/v) FCS, 1 % (v/v) Pen/Strep; LLC-PK1: M199, 5 % (v/v) FCS, 1 % (v/v) Pen/Strep, 2 mM Glutamin). The next day, 6 µg of plasmid DNA was diluted in 687.5 µl NaCl (150 mM), 1 mM Polyethylenimine was added and incubated for 10 min at rt. The transfection mix was slowly pipetted onto the cells under constant rocking of the dish. Cells were incubated for 48 h without medium change at 37°C, 5 % CO₂. Analysis of results proofed difficult due to variable transfection efficiencies obtained with the methods used. Therefore, transfected cells were sorted to enrich GFP-positive cells. In brief, cells were washed with PBS, detached with Trypsin-EDTA, resuspended in culture medium and centrifuged at 300 xg, 5 min. Pelleted cells were resuspended in 500 µl MACS Buffer (PBS, 1 % (v/v) FCS, 5 mM EDTA) and a small aliquot was taken. Non-viable cells were removed by FSC, SSC and staining with propidium iodide, while a FACS Aria II cell sorter (BD Biosciences) was used to sort the remaining cells. Unsorted as well as GFP-negative and GFP-positive cells were pelleted (300x*g*, 5 min) and used for protein isolation and immunoblot as described above.

### 5.2 Testing of miGRK2 sequences in human and porcine cells

In total, five miGRK2 sequences were tested for knockdown efficiency *in vitro* (Fig. 5 A). Out of these five sequences, three showed a reduction of GRK2 on protein level, with miGRK2 503 and 794 achieving the highest reduction (-60 % and -30 % compared to miR neg ctrl, respectively). All other generated miGRK2 sequences did not result in a significant reduction of GRK2 *in vitro.*

The sequence miGRK2 794 was designed to target human and porcine GRK2 *in vitro.* Therefore, LLC-PK1 cells were transfected with a plasmid encoding miR neg ctrl. or miGRK2 794. Cells were then sorted as described above and immunoblotted for GRK2, GFP and GAPDH (Fig. 5B, lower panel). These data indicate that miGRK2 794 induces a knockdown in LLC-PK1 cells. However, the data also suggest that this particular sequence is less efficient in a porcine cell line compared to human cells, which might be in part explained by the lower transfection efficiency compared to human cells.

Non-Standard literature cited:
Asokan (2012, Mol Ther 20(4):699
Bish et al. (2008), Human Gene Therapy 19:1359
Cannavo et al. (2013), Front Physiol 4, 264
Chung et al. 2006. Nucleic Acids Res. 34, e53
Evron et al. (2012), Trends Pharmacol. Sei. 33, 154-164
Greenberg et al. (2016), Lancet 387:1178
Müller et al. 2006. Cardiovasc. Res. 70, 70-78
Naso et al. (2017), BioDrugs 31:317
Raake et al., (2008), Eur. Heart J. 34, 1437-1447
Rockman et al., 1991, Proc. Natl. Acad. Sci. 88: 8277-8281
Rockman et al., 1994, Proc. Natl. Acad. Sci. 91(7): 2694-2698
Ungerer et al, (1993), Circulation 87, 454-463
Zeng, Y., and Cullen, B.R. 2003. RNA 9, 112-123

## Claims

1. A polynucleotide comprising and/or encoding a microRNA (miRNA) decreasing G protein-coupled receptor kinase 2 (GRK2) gene expression in a host cell.

2. The polynucleotide of claim 1, wherein said miRNA comprises, preferably consists of, a nucleic acid sequence (miRNA sequence) being the reverse complement of a GRK2 target nucleic acid sequence (GRK2 target sequence), preferably wherein said GRK2 target sequence consists of at least 15, preferably of from 18 to 22, more preferably 21, contiguous nucleotides of a nucleic acid sequence at least 70% identical to a GRK2 encoding polynucleotide, preferably a GRK2 encoding mRNA.

3. The polynucleotide of claim 1 or 2, wherein said GRK2 target sequence comprises, preferably consists of, the sequence of any one of SEQ ID NO:s:6 to 10.

4. The polynucleotide of claim 2 or 3, wherein said polynucleotide comprises and/or encodes said miRNA sequence, a spacer sequence, and a passenger sequence, preferably in a 5' to 3' order, wherein said passenger sequence is the reverse complement of said miRNA sequence but lacks at least one nucleotide corresponding to a position of from 4 to 12 of said reverse complement of said miRNA sequence.

5. The polynucleotide of any one of claims 1 to 4, wherein said miRNA consists of a nucleic acid sequence selected from SEQ ID NOs:1 to 5 or a sequence at least 80% identical thereto; preferably consists of a nucleic acid sequence selected from SEQ ID NOs:1 to 5.

6. The polynucleotide of any one of claims 1 to 5, wherein said polynucleotide comprises nucleic acid sequences of (i) SEQ ID NOs:1 and 11, (ii) SEQ ID NOs:2 and 12, (iii) SEQ ID NOs:3 and 13, (iv) SEQ ID NOs:4 and 14, (v) SEQ ID NOs:5 and 15, or (vi) any combination of (i) to (v).

7. The polynucleotide of any one of claims 1 to 6, wherein said polynucleotide comprises a nucleic acid sequence selected from SEQ ID NOs:16 to 20 or a sequence at least 80% identical thereto; preferably consists of a nucleic acid sequence selected from SEQ ID NOs:16 to 20.

8. The polynucleotide of any one of claims 1 to 7, wherein said polynucleotide comprises, preferably consists of, the nucleic acid sequence of any one of SEQ ID NOs:21 to 25, or a sequence at least 80% identical thereto, preferably comprises the nucleic acid sequence of any one of SEQ ID NOs: 21 to 25.

9. The polynucleotide of any one of claims 1 to 8, wherein said polynucleotide comprises or encodes at least one further miRNA.

10. The polynucleotide of any one of claims 1 to 9, wherein said polynucleotide is comprised in a vector packaging construct, preferably a viral vector packaging construct, more preferably an adeno-associated virus (AAV) packaging construct, preferably an AAV9 packaging construct.

11. A vector comprising the polynucleotide according to any one of claims 1 to 10.

12. A polynucleotide according to any one of claims 1 to 10, and/or a vector according to claim 11 for use in medicine.

13. A polynucleotide according to any one of claims 1 to 10, and/or a vector according to claim 11 for use in treatment and/or prevention of heart failure in a subject, preferably chronic heart failure, hypertrophic cardiomyopathy, dilatative cardiomyopathy, ischemic cardiomyopathy, and/or coronary heart disease.

14. A device comprising the polynucleotide according to any one of claims 1 to 10, and/or a vector according to claim 11.

15. A kit comprising the polynucleotide according to any one of claims 1 to 10, and/or a vector according to claim 11, comprised in a housing.
